# EUROPEAN PATENT APPLICATION

(11) **EP 3 789 768 A1**
(43) Date of publication of application: **10.03.2021**
(21) Application number: 19196074.9
(22) Date of filing: 07.09.2019
(51) Int. Cl.: G01N 33/563

(54) **METHOD FOR DETERMINING THE EFFECTIVENESS OF A NANOPARTICLE-BASED THERAPY**

(71) Applicant: Universitätsklinikum Hamburg-Eppendorf, 20246 Hamburg (DE)
(72) Inventor: CARAMBIA, Antonella, 20259 Hamburg (DE); HERKEL, Johannes, 22393 Hamburg (DE); KRZIKALLA, Daria, 22529 Hamburg (DE)
(74) Representative: Uexküll & Stolberg

(57) **Abstract**

The present invention relates to a method for determining the effectiveness of a nanoparticle-based therapy for suppressing an immune response in a subject. More specifically, the method comprises the determination of the expression levels of one or more marker nucleotide sequences in a sample from the subject and comparing said expression level to the expression level of the same nucleotide sequence obtained from a control sample. An increased expression level of the at least one nucleotide sequence relative to the control indicates that the nanoparticle-based therapy is effective in said subject.

## Description

The present invention relates to a method for determining the effectiveness of a nanoparticle-based therapy for suppressing an immune response in a subject. More specifically, the method comprises the determination of the expression level of one or more marker nucleotide sequences in a sample from the subject and comparing said expression level to the expression level of the same nucleotide sequence obtained from a control sample. An increased expression level of the at least one nucleotide sequence relative to the control indicates that the nanoparticle-based therapy is effective in said subject.

### BACKGROUND OF THE INVENTION

Regulatory T lymphocytes (Tregs) have been found to be important mediators of immune tolerance to self antigens, and it has been suggested that the inactivation of these cells is the main cause for the development of autoimmune diseases. It has been demonstrated that Treg-mediated suppression of autoimmune diseases can be achieved in vivo by taking advantage of the ability of the liver to promote immune tolerance (Lüth et al. (2008), J. Clin. Invest. 118: 3403-3410).

Specifically, it was shown that the expression of the neural autoantigen myelin basic protein (MBP) in the liver of transgenic mice induced protection from autoimmune neuroinflammation in a multiple sclerosis model. Protection from autoimmunity was mediated by MBP-specific CD4⁺CD25⁺Foxp3⁺ Tregs, as demonstrated by the ability of these cells to prevent diseases when adoptively transferred into non-transgenic mice. It was concluded from these results that autoantigen expression in the liver may generate autoantigen-specific Tregs that suppress the autoimmune disease.

Another possibility to suppress autoimmune diseases resides in the delivery of autoantigens to the liver via nanoparticles. WO 2013/072051 describes a novel type of water-soluble nanoparticles that carry autoantigen peptides on their outer surface and which provide for the targeted delivery of these peptides to liver sinusoidal endothelial cells (LSEC). The nanoparticles selectively accumulate in LSEC. It is described that the nanoparticles induce the generation of CD4⁺CD25⁺Foxp3⁺ Tregs that effectively suppress the undesired immune reaction. Accordingly, it discloses a nanoparticle-based therapy for suppressing an immune response in a subject.

While the treatment approach using the nanoparticles of WO 2013/072051 is safe, reliable and highly effective, it would be desirable to have a diagnostic tool to predict, determine and monitor the effectiveness of the nanoparticle-based therapy. Such diagnostic tool would be particularly helpful to determine the therapy plan for an individual patient in terms of nanoparticle dosis and frequency of administration. The present invention provides a method that allows determining the effectiveness of a nanoparticle-based therapy as described in WO 2013/072051 based on a certain set of biomarkers.

### DESCRIPTION OF THE INVENTION

The present invention is based on the insight that the administration of nanoparticles as described in WO 2013/072051 results in the up-regulation of certain genes and their products, including cytokines, chemokines and co-inhibitory receptors in the subject that receives the nanoparticles.

This insight opens up the possibility to use these marker genes, and the proteins encoded by these genes, for monitoring the effectiveness of treatment after administration of the nanoparticles to the subject. In addition, it renders possible to enhance the effectiveness of treatment by administering certain cytokines, chemokines and/or activating ligands for co-inhibitory receptors together with the nanoparticles.

According to a first aspect, the present invention therefore provides a method for determining the effectiveness of a nanoparticle-based therapy for suppressing an immune response in a subject, said method comprising
(a) providing a test sample from a subject to whom nanoparticles have been administered to suppress an immune response;
(b) determining in said test sample the expression level of at least one nucleotide sequence selected from the group consisting of SEQ ID NOs:1-9 or the complement thereof; and
(c) comparing the expression level of the nucleotide sequence determined in the test sample to the expression level of the same nucleotide sequence determined in a control sample,
wherein the detection of an increased expression level of the at least one nucleotide sequence in the test sample relative to the control sample indicates that the nanoparticle-based therapy is effective in said subject.

The subject to be tested by the method of the present invention has been subjected to nanoparticle-based therapy for suppressing an immune response which means that nanoparticles have been administered to the subject which are suitable for ameliorating or suppressing an undesired immune response, such as an immune response that occurs in connection with an autoimmune disease or chronic inflammation.

It is particularly preferred that the nanoparticle-based therapy is performed with nanoparticles as described in WO 2013/072051. Accordingly, the nanoparticles that have been administered to the subject comprise
(a) a micelle comprising an amphiphilic polymer rendering the nanoparticle water-soluble, and
(b) a peptide comprising the at least one T cell epitope associated with the outside of the micelle.

As described in WO 2013/072051, the nanoparticles accumulate in the liver, in particular, in liver sinusoidal endothelial cells, after in vivo administration to a subject. If the nanoparticles are loaded with a peptide, they induce regulatory T cells which are specific to the peptide. For example, if the peptides which are loaded onto the nanoparticles are derived from the Myelin Basic Protein (MBP), regulatory T cells are induced that are capable of suppressing experimental autoimmune encephalitis (EAE) in an animal model.

The nanoparticles have a diameter which ranges from about 1 to about 999 nm, and preferably from 2 to about 600 nm, from about 5 to about 500 nm, from about 10 to about 300 nm, from about 30 to about 100 nm, from 40 to about 50 nm, and most preferably from about 30 to about 50 nm. The nanoparticles may be negatively charged or uncharged. Preferably, the nanoparticles are negatively charged. The polymer coating of the nanoparticle may comprise acid groups, e.g. carboxylic acid groups, which lead to a negative charge.

The micelle of the nanoparticles comprises an amphiphilic polymer that renders the nanoparticle water-soluble. The term "micelle" relates to an aggregate of amphiphilic molecules dispersed in an aqueous solution. The hydrophilic parts of the amphiphilic molecules are in contact with the surrounding solvent, sequestering the hydrophobic "tail" regions of the amphiphilic molecules on the inside of the micelle, and thus render the nanoparticle water-soluble. This type of micelle is known as a normal phase micelle or oil-in-water micelle. Preferably, the micelle is formed by a single layer of the amphiphilic polymer.

The amphiphilic polymer forming the micelle preferably is a synthetic polymer. It may comprise a hydrophobic region comprising a hydrophobic "tail" having a length of about 14-22, preferably, 16-18 carbon atoms. The hydrophilic region of the polymer may be negatively charged in an aqueous solution. The molecular mass of the polymer may be about 30,000m to 50,000 g/mol. Preferably, the polymer is a maleic copolymer, such as poly(maleic anhydride-alt-1-octadecene). The polymer may also be poly(maleic anhydride-alt-1-tetradecene) or polyisoprene-block polyethyleneoxide block copolymer (PI-b-PEO). The micelle may be formed by one or more than one, e.g., two, three or four amphiphilic molecules polymers.

In one embodiement, the core of the micelle does not comprise additional molecules or compounds, but consists only of the hydrophobic regions of the amphiphilic polymers. In another embodiment, the micelle coats a solid hydrophobic core, e.g. an inorganic core. The inorganic core preferably may be a traceable inorganic core, comprising e.g. iron oxide, CdSe, silver or gold. The diameter of the core preferably is from about 2 to about 500 nm, from about 5 to about 30 nm, and more preferably from about 7 to about 12 nm.

The nanoparticles may be associated with a peptide that is covalently linked to the outside of the micelle. The peptide may also be non-covalently associated to the nanoparticle. Such nanoparticles may be prepared, e.g. by dipping and drying. The peptide may comprise from about 8 to about 2000 amino acids, from about 8 to about 200 amino acids, from about 8 to about 100 amino acids, from about 9 to about 60 amino acids, or from about 10 to about 20 amino acids.

The peptide may be synthesized, recombinantly expressed or isolated from a natural source. The peptide, or at least the epitope against which regulatory T cells are to be generated, is preferably derived from a peptide/protein against which an inflammatory immune response is to be suppressed, e.g., in the context of treatment or prevention of an autoimmune disease or an allergy. The peptide may, e.g., be an allergen, an autoimmune antigen, or a fragment or derivative thereof. The peptide can combine various epitopes from various antigens. Fusion polypeptides comprising peptides from more of one source may be used as well.

The peptide comprises at least one T cell epitope to which regulatory T cells are to be generated. At least one epitope needs to be capable of being presented by cells of the subject to which the nanoparticles are to be administrated, i.e., the peptide and/or the subject need to be appropriately selected. Preferably, the peptide comprises several epitopes which enable it to be presented in a plurality of Major Histocompatibility Complex types.

In the first step of the method of the invention, a test sample is provided. The test sample has been obtained from the subject that needs to be tested for therapy effectiveness, i.e. the subject to whom nanoparticles were administered to suppress an immune response. The sample can be in principle any kind of sample which allows the detection of the expression levels of the marker genes and proteins defined elsewhere herein. In a preferred embodiment, the test sample is a blood sample, such as a serum sample. In another preferred embodiment, the test sample is a tissue sample, such as a liver tissue biopsy. The method of the invention can be performed with samples from all different types of mammals. Preferably, however the test sample is derived from a human subject.

In a preferred embodiment, the subject is suffering from a disease or condition that is characterized by an undesired immune response, which means that the immune response contributes to the disease or condition and is adverse to the health state of the subject. The disease or condition may be an autoimmune disease, an allergy or an inflammatory disease, such as a chronic inflammatory disease. It is particularly preferred that the sample is derived from a subject suffering from an autoimmune disease selected from the group consisting of multiple sclerosis, type I diabetes, rheumatoid arthritis, systemic lupus erythematosus, ulcerative colitis, Crohn's disease, autoimmune cardiomyopathy, autoimmune hepatitis, Graves' disease, Guillain-Barre syndrome (GBS), Hashimoto's thyroiditis, idiopathic thrombocytopenic purpura, juvenile idiopathic arthritis, myasthenia gravis, pemphigus vulgaris, psoriasis, Reiter's syndrome, scleroderma, Sjögren's syndrome, vasculitis, vitiligo, and Wegener's granulomatosis.

If the test sample is a blood sample, the sample may be processed before expression level measurement. For example, erythrocytes that would otherwise potentially interfere with expression level measurement can be removed from the sample, e.g. by separating them from the other cells included in the test sample or by lysing them with a suitable agent such as ammonium chloride potassium lysis buffer. In addition, heparin or another anticoagulant can be added to the test sample to avoid blood coagulation.

Where necessary, the T cell fraction can be enriched before expression level measurement. For example, T cells that have been stimulated with nanoparticles associated with a peptide, e.g. an autoantigen peptide, can be enriched via activation markers like CD154 for Effector T cells and CD137 for Tregs. Such T cells can be separated from other cells by fluorescence-activated cell sorting (FACS) or magnetic-activated cell sorting (MACS).

In a subsequent step of the method of the invention, the expression level of at least one nucleotide sequence selected from the group consisting of SEQ ID NOs:1-9 or the complement thereof is determined in said test sample. The sequences provided in SEQ ID NOs:1-9 refer to the mRNA sequences of genes encoding cytokines and/or chemokines and/or co-inhibitory receptors which have been found to be up-regulated after administration of the nanoparticles to the subject. These genes are referred to as the "marker genes" herein below. The corresponding expression products, i.e. the proteins encoded by the nucleotide sequences provided in SEQ ID NOs:1-9, are referred to as the "marker proteins" herein below. SEQ ID NO:1 refers to the mRNA sequence encoding the human "cytotoxic T-lymphocyte associated protein 4 (CTLA4)". Further information on this sequence is available in the NCBI databases under Gene ID: 1493 (NG_011502.1). SEQ ID NO:2 refers to the mRNA sequence encoding the human "T cell immunoreceptor with Ig and ITIM domains (TIGIT)". Further information on this sequence is available in the NCBI databases under Gene ID: 201633. SEQ ID NO:3 refers to the mRNA sequence encoding the human "hepatitis A virus cellular receptor 2 (HAVCR2, also known as TIM3)". Further information on this sequence is available in the NCBI databases under Gene ID: 84868 (NG_030444.1). SEQ ID NO:4 refers to the mRNA sequence encoding the human "lymphocyte activating 3 (LAG3)" protein. Further information on this sequence is available in the NCBI databases under Gene ID: 3902. SEQ ID NO:5 refers to the mRNA sequence encoding the "human programmed cell death 1 (PDCD1 also known as PD-1)" protein. Further information on this sequence is available in the NCBI databases under Gene ID: 5133 (NG_012110.1). SEQ ID NO:6 refers to the mRNA sequence encoding "interferon gamma (IFN-γ)" protein. Further information on this sequence is available in the NCBI databases under Gene ID: 3458 (NG_015840.1). SEQ ID NO:7 refers to the mRNA sequence encoding "C-X-C motif chemokine ligand 9 (CXCL9)". Further information on this sequence is available in the NCBI databases under Gene ID: 4283. SEQ ID NO:8 refers to the mRNA sequence encoding "C-X-C motif chemokine receptor 3 (CXCR3)". Further information on this sequence is available in the NCBI databases under Gene ID: 2833 (NG_029076.1). SEQ ID NO:9 refers to the mRNA sequence encoding "Interleukin 10". Further information on this sequence is available in the NCBI databases under Gene ID: 3586 (NG_012088.1).

According to a preferred embodiment, step (b) of the method of the invention comprises determining the expression level of more than one gene, for example 2, 3, 4, 5, 6, 7, 8 or 9 genes selected from the group consisting of SEQ ID NOs:1-9. According to a particularly preferred embodiment, step (b) of the method of the invention comprises determining the expression level of all genes from the group of the genes shown in SEQ ID NOs:1-9.

It is preferred to use defined combinations of the described marker genes for determining the effectiveness of a nanoparticle-based therapy for suppressing an immune response. For example, it is preferred that the method of the invention at least comprises determining the expression level of the following genes: SEQ ID NOs: 1 and 2; SEQ ID NOs: 1, 2 and 3; SEQ ID NOs: 1, 2, 3 and 4; SEQ ID NOs: 1, 2, 3, 4 and 5; SEQ ID NOs: 1, 2, 3, 4, 5 and 6; SEQ ID NOs: 1, 2, 3, 4, 5, 6 and 7; SEQ ID NOs: 2 and 3; SEQ ID NOs: 2, 3 and 4; SEQ ID NOs: 2, 3, 4 and 5; SEQ ID NOs: 2, 3, 4, 5 and 6; SEQ ID NOs: 2, 3, 4, 5, 6 and 7; SEQ ID NOs: 2, 3, 4, 5, 6, 7 and 8; SEQ ID NOs: 3 and 4; SEQ ID NOs: 3, 4 and 5; SEQ ID NOs: 3, 4, 5 and 6; SEQ ID NOs: 3, 4, 5, 6 and 7; SEQ ID NOs: 3, 4, 5, 6, 7 and 8; SEQ ID NOs: 4 and 5; SEQ ID NOs: 4, 5 and 6; SEQ ID NOs: 4, 5, 6 and 7; SEQ ID NOs: 4, 5, 6, 7 and 8; SEQ ID NOs: 5 and 6; SEQ ID NOs: 5, 6 and 7; SEQ ID NOs: 5, 6, 7 and 8; SEQ ID NOs: 6 and 7; SEQ ID NOs: 6, 7 and 8; SEQ ID NOs: 7 and 8; SEQ ID NOs: 6, 7 and 9; SEQ ID NOs: 6, 7, 8 and 9; SEQ ID NOs: 7, 8 and 9; SEQ ID NOs:8 and 9; SEQ ID NOs:1, 6, 8 and 9. According to a particular embodiment, the methods of the invention include the determination of the expression levels of all of the above marker genes.

The detection of the expression level of the one or more marker genes can be conducted in accordance with routine methods known in the art. The determination of the expression of the marker genes can be performed either at the transcriptional (mRNA) level or at the translational (protein) level.

In a preferred embodiment, the method of the invention is performed such that the expression level of at least one nucleotide sequence set forth in SEQ ID NOs:1-9 is determined at the transcriptional level. Suitable methods for monitoring gene expression at the transcriptional level include those which allow for the quantitative or semi-quantitative detection of mRNA levels, for example, quantitative RT-PCR (e.g., TaqMan™ RT-PCR), real-time RT-PCR, Northern-Blot analysis or other methods. For example, the mRNA can be isolated from the cells contained in the test sample, preferably from the blood sample. Typically, the total RNA is isolated from the sample in a first step. Methods for isolating total RNA are well known in the art and are extensively discussed in the literature (see, for example, Greene & Sambrook (2012), Molecular Cloning - A Laboratory Manual, 4 ed., Cold Spring Harbor Laboratory, Cold Spring Harbor, New York). Methods for isolating RNA regularly involve the lysis of the cells of the respective tissue samples. Cell lysis may be performed by use of detergents which are capable of disrupting the plasma membrane of the cells. For example, buffers containing guanidine thiocyanate or SDS may be used for cell lysis. The methods may also comprise steps in which the DNA of the cells is enzymatically digested in order to obtain pure RNA without traces of DNA which might interfere with the further downstream applications. Inhibitors of enzymes which lead to the degradation of the RNA may also be added to the lysis buffer. Kits for the isolation of highly purified total RNA are also available from several manufactures. For example, the EZ1 RNA Cell Kits of Qiagen (Hilden, Germany) can be readily used for RNA isolation and purification. Similar kits are available also from a number of other manufacturers.

The total RNA isolated from the test sample will normally comprise different types of RNA. Preferably, the total RNA comprises mRNA, transfer RNA (tRNA) and ribosomal RNA (rRNA). For the methods of the present invention, it is desirable to enrich the mRNA fraction with respect to fractions of other cellular RNAs. Preferably, the mRNA is separated from other RNA molecules. Methods for enriching or purifying mRNA are known in the art. For example, an affinity chromatography using oligo(dT) or poly(U) coupled to a solid matrix, such as a cellulose or Sephadex™ matrix can be performed, as most mRNAs contain a poly(A) tail at their 3' terminus. The separation of mRNAs from total RNA can also be performed using a commercial kit like the Oligotex mRNA Mini Kit of Qiagen (Hilden, Germany).

The mRNA is subsequently used for the determination of the expression level of one or more nucleotide sequences selected from the group of genes shown in SEQ ID NOs:1-9. Preferably, the determination of the expression level of the respective marker genes is performed simultaneously, for example by use of a microarray (see below).

Several methods for determining the expression level of genes are known in the art. In a preferred embodiment of the invention, the expression level of the selected genes is determined by use of a microarray. The term "microarray" refers to the orderly arrangement of spatially resolved probes, for example nucleic acid probes, on a substrate. The substrate is preferably a solid substrate having a surface with a plurality of probes attached in different known locations (spots). The spots on a microarray are normally printed on the microarrays or synthesized by photo-lithography or by ink-jet printing. On a common microarray, there may be thousands of spots, wherein each of the spots may contain a high number of identical probes, such as nucleic acid fragments or oligonucleotides. Such microarrays typically have a density of at least 100 oligonucleotides or fragments per cm². In certain embodiments the arrays can have a density of about at least 500, at least 1000, at least 10000, at least 10⁵, at least 10⁶, at least 10⁷ oligonucleotides or fragments per cm². The support can be a glass slide or membrane on the surface of which the probes are attached at fixed locations or spots.

Preferably, the probes of the microarray to be used in the diagnostic method of the invention are nucleic acid probes, i.e. oligonucleotides of fragments of DNA or RNA. The probes may be native DNA or RNA molecules or analogues of DNA or RNA or portions thereof. The length of the nucleic acid fragments or oligonucleotides may vary from 10-500 bp and is preferably in the range of 15, 20, 25, 30, 35, 40, 45 or 50 bp. The probe for a particular gene may even correspond to the full length coding sequence of said gene. Oligonucleotide probes may be conveniently synthesized by DNA synthesizer according to the well-known phosphoramidite method. The probes may be modified, for example by synthesizing oligonucleotides which have a modified backbone. Such modifications are known in the art and comprise, for example, phosphorothioate or phosphoramidate modifications of the backbone. The probe may also comprise one or more modified sugar moieties.

For the purposes of the present invention, the nucleic acid probes of the microarray used in the method of the invention are derived from the sequences provided in SEQ ID NOs:1-9. The probes are complementary and hybridizable to mRNA derived from the expression of one or more of the genes shown in SEQ ID NOs:1-9. Any sequence which is derived from one of the genes of SEQ ID NOs:1-9 and which is suitable for being used as a probe on a microarray can be used. As used herein, a probe is "derived" from one of the genes shown in SEQ ID NOs:1-9, if the nucleotide sequence of the probe is comprised by said gene sequence, whereas substitutions, insertions or deletions of the probe sequence are possible, as long as the probe remains hybridizable to the mRNA of the respective gene. Thus, the probe sequence will usually share a high degree of identity to a sequence stretch of the respective gene. Preferably, the probe will exhibit an identity with the sequence stretch of the respective gene of 80%, more preferably 85%, 90%, 95% or 99% or more over a stretch of at least 15 nucleotides. Methods and criteria for selecting probe sequences are well known in the art.

The present invention further provides a microarray for determining the effectiveness of a nanoparticle-based therapy for suppressing an immune response in a subject which comprises the probes derived from the above-recited marker genes. The microarray consists of nucleic acid probes from not more than 1500 different genes, preferably human genes. It is preferred that the microarray of the invention consists of nucleic acid probes from not more than 1000 different genes, preferably not more than 900, 800, 700, 600, 500, 400, 300, 200, 100 or 50 different genes. Further, the microarray of the invention comprises nucleic acid probes derived from at least one or more of the nucleotide sequences provided in SEQ ID NOs:1-9. The microarray may also comprise nucleic acid probes suitable for the normalization of gene expression. Such probes are well known in the field and comprise, for example, probes derived from housekeeping genes such as S27a or L19. Different methods may be used for normalization of gene expression. After elimination of genes below background levels, datasets can be normalized by routine methods.

According to a further aspect, the invention relates to the use of a microarray as described above for determining the effectiveness of a nanoparticle-based therapy for suppressing an immune response in a subject.

Alternatively, commercially available microarrays which comprise probes for measuring the expression of one or more of the genes referred to in SEQ ID NOs:1-9 may also be used for the methods of the invention. Several different microarrays for monitoring the mRNA levels of multiple genes are known in the art. For example, the Codelink™ Human 20K Bioarray (GE Healthcare) may be used for performing the methods of the present invention. This array system contains about 20.000 gene specific probes which are optimized for hybridization reaction with complementary RNA molecules. Other microarray systems as well as suitable imaging equipment for reading out the results of the hybridization reaction are commercially available.

In another preferred embodiment of the invention, the expression level of the selected genes is determined by use of a reverse transcription PCR analysis. For example, quantitative RT-PCR (qRT-PCR) can be used to determine the expression level of the one or more marker gene. In this approach, the mRNA obtained from the tissue sample will be subjected to reverse transcription, and the resulting cDNA will be exponentially amplified in a PCR reaction. Primers which are specific for a particular sequence, random hexamers, or oligo-dT primers may be used for priming in the reverse transcription step. The derived cDNA can then be used as a template in the subsequent PCR reaction.

In a preferred embodiment of the invention, quantitative RT-PCR may be performed as quantitative real time RT-PCR. As will be understood by those skilled in the art, real time PCR methods provide for the quantification of the target DNA after each round of amplification. Quantification is typically achieved by use of fluorescent dyes or oligonucleotide probes which comprise fluorescently labeled moieties. The methods are designed such that fluorescence increases proportionally with the amount of PCR product obtained from the amplification reaction.

In a simple procedure of RT-PCR, quantitative real time RT-PCR may be performed by use of DNA intercalating dyes, such as SYBR® Green I. The fluorescent signal of the dye which has intercalated into the DNA produced in the amplification reaction can be detected. The use of DNA intercalating dyes, however, cannot detect DNA specifically, so that it does not allow drawing any conclusion as to whether the amplified DNA in fact corresponds to the target sequence. Thus, according to a more preferred embodiment, the methods for quantitative real time RT-PCR allow for the detection of the specific product obtained by the amplification reaction. For this purpose, sequence specific probes can be used which are labeled with groups or moieties which can be quantitatively detected via Förster resonance energy transfer (FRET). Examples for such probes are TaqMan® probes, Molecular Beacons, Scorpion probes, LightCycler probes and the like.

The invention further provides kits for performing the methods contemplated by the invention at a transcriptional level. According to one embodiment, a kit for determining the effectiveness of a nanoparticle-based therapy for suppressing an immune response in a subject by microarray technique is provided which comprises a microarray as described herein. The kit may further comprise reagents and other means for microarray hybridization and detection. For example, the kit may comprise buffers and enzymes for first strand synthesis, cDNA purification and in vitro transcription and labeling. Further it may comprise buffers for hybridization, washing and cRNA fragmentation. It may also contain instructions for using the microarray.

According to a further embodiment, a kit for determining the effectiveness of a nanoparticle-based therapy for suppressing an immune response in a subject by RT-PCR is provided. The RT-PCR may comprise primers for amplifying two or more genes selected from the group of genes shown in SEQ ID NOs:1-9. It may also comprise buffers, polymerase enzymes for reverse transcription and amplification and probes, such as probes which are labeled with fluorescent groups suitable to be used in FRET-based techniques. Of course, the kit may also comprise instructions as to the use of the components in quantitative RT-PCR.

In another preferred embodiment, the method of the invention is performed such that the expression level of the at least one nucleotide sequence is determined at the translational level. In such an embodiment, step (b) of the above method comprises determining in the test sample the protein level of at least one protein encoded by a nucleotide sequence selected from the group consisting of SEQ ID NOs:1-9 or the complement thereof. The sequences of the corresponding marker proteins which are encoded by the marker genes are set forth in SEQ ID NOs:10-18.

Accordingly, the invention also relates to a method for determining the effectiveness of a nanoparticle-based therapy for suppressing an immune response in a subject, said method comprising
(a) providing a test sample from a subject to whom nanoparticles as described above have been administered to suppress an immune response;
(b) determining in said test sample the amount of at least one amino acid sequence selected from the group consisting of SEQ ID NOs:10-18; and
(c) comparing the amount of the amino acid sequence determined in the test sample to the amount of the same amino acid sequence determined in a control sample,
wherein the detection of an increased amount of the at least one amino acid sequence in the test sample relative to the control sample indicates that the nanoparticle-based therapy is effective in said subject.

Suitable methods for monitoring gene expression at the translational level include those which allow for the quantitative or semi-quantitative detection of the protein levels of the proteins encoded by the marker genes in the test sample. The protein levels may be determined by any suitable method which is able to specifically detect the expression products of at least one nucleotide sequence set forth in SEQ ID NOs:1-9 in the test sample. The detection of the protein may be based on a molecule that specifically binds to the marker protein or the separation of the marker protein from other proteins that are present in the sample.

Molecules that specifically bind to the marker protein include antibodies and antibody fragments with binding activity for the marker proteins. Such antibodies or fragments thereof may be used for detection of the proteins in a flow cytometry, Western blot, enzyme-linked immunosorbent-assay (ELISA), polarization analysis, (quantitative) surface plasmon resonance (SPR) or in an immunohistochemical method, including quantitative electronmicroscopic methods. Other methods which are able to detect specific binding include Förster fluorescence resonance energy transfer (FRET). Methods which allow the quantitative detection of the proteins by separating the protein from other components in the biological sample include quantitative mass spectrometric methods, electrophoretic methods, such as two-dimensional gel electrophoresis, and chromatographic methods, such as size-exclusion chromatography or ion-exchange chromatography.

For example, flow cytometry can be used to determine the protein levels in the test sample. In this approach, the cells in the test sample will be subjected to staining with specific antibodies that can be detected directly or indirectly using fluorescence or mass spectrometry. Antibodies which are specific for a particular protein sequence set forth in SEQ ID NOs:10-18 can be directly labeled with fluorochromes or isotopically pure metal elements and may be used for detection in fluorescence-based flow cytometers or mass spectrometry-based cytometers by time of flight. The fluorescence or metal signals increase proportionally with the amount of specific antibody bound to each cell within the test sample. Alternatively, the binding of antibodies which are specific for a particular protein sequence set forth in SEQ ID NOs:10-18 can be indirectly quantified by detection with fluorochrome- or metal-labeled secondary antibodies. In another alternative, antibodies which are specific for a particular protein sequence derived from one of the marker proteins set forth in SEQ ID NOs:10-18 can be conjugated to biotin, and the cell binding can be quantified using Avidin or Streptavidin conjugates with fluorochromes or metals.

In a simple flow cytometry procedure, protein quantification is achieved by use of specific antibodies labeled with fluorochromes, such as Fluorescein (FITC), R-Phycoerythrin (PE), or allophycocyanin (APC) and the like. The fluorescent signal of the fluorochrome which is conjugated to the specific antibody is then detected in a fluorescence-based flow cytometer. Suitable antibodies are available from various vendors.

If the detection of more than one of the marker proteins in the test sample is desired, combinations of different antibodies are used. Such combinations will normally comprise differently labeled antibodies so as to allow all marker protein levels independently from each other.

It is preferred to use defined combinations of antibodies specific for the described marker proteins in combination with additional methods that provide detection of antigen-specific lymphocytes. These methods include the use of fluorochrome- or metal-conjugated peptide-loaded MHC multimers (see for example Davis et al. (2011), Nat Rev Immunol, 11:551-8), or the use of lymphocyte activation markers for detection or enrichment of lymphocytes after antigen-specific stimulation (see for example Bacher et al. (2013), J Immunol, 190:3967-76).

A kit for determining the effectiveness of a nanoparticle-based therapy for suppressing an immune response in a subject by flow cytometry is also provided. The cytometry kit may comprise fluorochrome-labeled or metal-labeled antibodies for detecting one or more protein sequences set forth in SEQ ID NOs:10-18. It may also comprise MHC multimers, buffers, or reagents for intracellular protein staining suitable to be used in fluorescence-based or time of flight-based flow cytometry techniques. Of course, the kit may also comprise instructions as to the use of the components in flow cytometry.

Once the expression level of the at least one nucleotide sequences shown in SEQ ID NOs:1-9 in the test sample has been determined, it is compared to the expression level of the same nucleotide sequence that has been determined in a control sample. An increased expression level of the at least one nucleotide sequence in the test sample relative to the control sample indicates that the nanoparticle-based therapy is effective in said subject. The control sample is preferably derived from the same subject at a time-point before treatment with nanoparticles. Alternatively, the control sample can be derived from a subject that has not been treated with nanoparticles. The subject may be a healthy subject that is not afflicted with any disease or pathological condition. Alternatively, the subject from whom the control sample is obtained may suffer from the same disease or condition as the subject that shall be tested by the method of the invention.

Hence, if a test subject exhibits a substantially increased expression level for at least one nucleotide sequences shown in SEQ ID NOs:1-9 in the test sample relative to the control sample, this indicates that the nanoparticle-based therapy is effective in said subject. According to the invention, the occurrence of a therapeutic effect is assumed if at least one nucleotide sequence shown in SEQ ID NOs:1-9 exhibits an expression level that is increased in the test sample relative to the control sample at least by 50%, at least by 100%, at least by 200%, at least by 300%, at least by 400%, at least by 500% or more. It is however more reliable to assume a therapeutic effect if more than one of the nucleotide sequence shown in SEQ ID NOs:1-9 exhibits an expression level that is increased in the test sample relative to the control sample at least by 50%, at least by 100%, at least by 200%, at least by 300%, at least by 400%, at least by 500% or more. It is particularly preferred that a therapeutic effect is assumed if at least 3, 4, 5, 6, 7, 8 or 9 of the nucleotide sequences shown in SEQ ID NOs:1-9 exhibit an expression level that is increased in the test sample relative to the control sample at least by 50%, at least by 100%, at least by 200%, at least by 300%, at least by 400%, at least by 500% or more.

In particular, if the expression level of the at least one nucleotide sequence selected from the group consisting of SEQ ID NOs:1-9 is determined by measuring protein amounts, the occurrence of a therapeutic effect is assumed if at least of the marker proteins is measured in the test sample in an amount that is increased at least by 50%, at least by 100%, at least by 200%, at least by 300%, at least by 400%, at least by 500% or more, relative to the control sample. It is however more reliable to assume a therapeutic effect if more than one of the marker proteins is measured in the test sample in an amount that is increased relative to the control sample at least by 50%, at least by 100%, at least by 200%, at least by 300%, at least by 400%, at least by 500% or more. It is particularly preferred that a therapeutic effect is assumed if at least 3, 4, 5, 6, 7, 8 or 9 of the marker proteins is measured in the test sample in an amount that is increased relative to the control sample at least by 50%, at least by 100%, at least by 200%, at least by 300%, at least by 400%, at least by 500% or more.

In yet another aspect, the invention relates to a method for determining a dosis of nanoparticles that is suitable for suppressing an immune response in a subject, said method comprising: (a) carrying out a method for determining the effectiveness of a nanoparticle-based therapy as describe herein above in a subject to whom nanoparticles have been administered in a certain dosis to suppress an immune response; and (b) adapting the dosis based on the expression levels determined in the test sample of the subject.

In yet another aspect, the invention relates to a method for assigning a subject to a treatment regimen of a nanoparticle-based therapy, said method comprising: (a) carrying out a method for determining the effectiveness of a nanoparticle-based therapy as describe herein above in a subject to whom nanoparticles have been administered in a certain dosis to suppress an immune response; and (b) assigning the subject to a treatment regimen based on the expression levels determined in the test sample of the subject.

In yet another aspect, the invention relates to a method for monitoring a nanoparticle-based therapy for suppressing an immune response in a subject, said method comprising (a) carrying out a method for determining the effectiveness of a nanoparticle-based therapy as describe herein above in a subject to whom nanoparticles have been administered in a certain dosis to suppress an immune response; and (b) assigning the subject to another dosis of nanoparticles if no increase in the expression levels in the test sample is observed.

The fact that the induction of tolerance is associated with an up-regulation of the co-inhibitory receptors LAG3, TIGIT, PD-1, HAVCR2 and CTLA4 demonstrates that the binding of ligands to these receptors must contribute to tolerance. Accordingly, the therapeutic effect of the administration of the nanoparticles can be enhanced by simultaneously administering one or more ligand of LAG3, TIGIT, PD-1, HAVCR2 and CTLA4. Ligands of these receptors are well known and have been extensively described in the art. For example, ligands of CTLA4 include CD80 (SEQ ID NO:19, Gene ID: 941) and CD86 (SEQ ID NO:20, Gene ID: 942). Ligands of PD-1 include CD274 (SEQ ID NO:21, Gene ID: 29126) and PDCD1LG2 (SEQ ID NO:22, Gene ID: 80380). Ligands of TIGIT include PVR (SEQ ID NO:23, Gene ID: 5817) and NECTIN2 (SEQ ID NO:24, Gene ID: 5819). Ligands of HAVCR2 include LGALS9 (SEQ ID NO:25, Gene ID: 3965) and HMGB1 (SEQ ID NO:26, Gene ID: 3146). Ligands of LAG3 include CLEC4G (SEQ ID NO:27, Gene ID: 339390).

Thus, in yet another aspect the invention relates to a pharmaceutical composition for use in a method of treating or preventing a disease that is associated with a specific immune response wherein suppression of said immune response is beneficial, wherein said composition comprises
(a) a nanoparticle as described above, i.e. a nanoparticle comprising a micelle comprising an amphiphilic polymer rendering the nanoparticle water-soluble, and a peptide comprising the at least one T cell epitope associated with the outside of the micelle, and
(b) one or more ligand of the receptors LAG3, TIGIT, PD-1, HAVCR2 and CTLA4.

The one or more ligand is preferably derived from one of the protein sequences shown in SEQ ID NO:10-27. This means that the ligand is either one of the proteins of SEQ ID NO:10-27 or a functional fragment derived from such a protein which is still capable of binding to its cognate receptor.

The ligands shown in SEQ ID NO:10-27 which normally occur as membrane-bound proteins on the surface of cells can be produced as recombinant proteins using routine techniques known in the art. For example, the ligands can be produced as recombinant fusion proteins in which the membrane-bound domain of the ligand is replaced by a domain of an immunoglobulin.

The pharmaceutical composition of the invention will comprise the one or more ligand in an amount that is suitable for enhancing the tolerance-inducing effect exerted by the nanoparticles. The specific amounts can be determined without any efforts in co-administration experiments in which, e.g., a fixed amount of nanoparticles is administered with increasing amounts of the one or more ligands, and the induction of tolerance in the subject is monitored.

In yet another aspect, the invention relates to a ligand that binds to a receptor selected from the group consisting of LAG3, TIGIT, PD-1, HAVCR2 and CTLA4 for use in a method of enhancing the therapeutic effect of a nanoparticle as described above, i.e. a nanoparticle comprising a micelle comprising an amphiphilic polymer rendering the nanoparticle water-soluble, and a peptide comprising the at least one T cell epitope associated with the outside of the micelle. The ligand preferably has an amino acid sequence set out in one of SEQ ID NO:10-27 or an amino acid sequence which is derived from one of those depicted in SEQ ID NO:10-27.

### BRIEF DESCRIPTION OF THE FIGURES

Fig. 1 shows that the administration of nanoparticles loaded with MBP peptide provides for a selective transport of the peptide to sinusoidal endothelial cells of the liver (LSEC), induces IFN-γ production by MBP-specific T cells in the liver (A), and leads to increased expression of CXCL9 (B) and CXCR3 (C) in the liver, and induces up-regulated expression of CTLA-4 by MBP-specific T cells in the liver (D).
Fig. 2 shows that inhibition of IFN-γ signal by anti-IFN-γ antibodies abolishes the nanoparticle-induced protection and leads to experimental autoimmune encephalomyelitis (EAE), shown as disease score over time (A) or the cumulated disease score (B).
Fig. 3 shows the results of measuring the expression levels of co-inhibitors in MBP-specific T cells in the blood in two models of hepatic tolerance, induced by MBP gene transfer (A) or MBP peptide-loaded nanoparticles (B), each on day 7 after immunization to MBP.
Fig. 4 shows the results of measuring the expression levels of co-inhibitors in MBP-specific T cells in the spleen in two models of hepatic tolerance, induced by MBP gene transfer (A) or MBP peptide-loaded nanoparticles (B), each on day 7 after immunization to MBP.
Fig. 5 shows that the administration of nanoparticles loaded with MBP peptide induces Interleukin-10 production by MBP-specific T cells in the liver (A) and in the blood (B), each on day 7 after immunization to MBP.

### EXAMPLES

The present invention will be described in the following by preferred embodiments which merely illustrate the invention, but should by no means limit the invention.

### Example 1: Preparation of nanoparticles

MBP-loaded nanoparticles were prepared by as described in WO 2013/072051. Briefly, oleic acid stabilized superparamagnetic iron oxide nanoparticles (SPIONs) were encapsulated into the amphiphilic polymer poly(maleic anhydride-alt-1-octadecene) according to the method of Freund et al. (2012), ACS Nano, 6:7318-25, and Heine et al. (2014), Beilstein J Nanotechnol., 5:1432-1440. Coupling of polymer coated nanoparticles to peptides was conducted in the presence of 1-Ethyl-3-(3-dimethylaminopropyl)-carbodiimide. A 150-fold excess of MBP peptide (Panatecs, Germany) was added and incubated for 2.5 h. Free peptide was removed under centrifugal force by using a spin filter (100 kDa, 4200 rpm, 25°C).

### Example 2: Measuring of IFN-γ and co-inhibitor expression levels in the liver

200 µg MBP peptide (Panatecs, Germany) in PBS were emulsified with complete Freund's adjuvant containing 4 mg/ml heat-killed *Mycobacterium tuberculosis,* strain H37RA (Difco, USA) and administered subcutaneously at the base of the tail to WT mice (B10.PL X B6.SJL/BoyJ). At the day of immunization and 48 h later, 200 ng pertussis toxin (Sigma-Aldrich) were administered intraperitoneally in 250 µl PBS. On the day of immunization, mice were injected intravenously with 150 µl of a solution containing MBP-loaded (20 µg peptide) or control nanoparticle (NP) of equal iron content. In order to study the antigen-specific T cell response, congenic CD4 T cells (CD45.1-) were isolated from spleens of tg4 mice via CD4 microbeads (Miltenyi Biotec). Five million MBP-specific CD4 T cells were adoptively transferred into CD45.1+ recipient mice one day after immunization. On day seven after immunization, liver non-parenchymal cells were isolated. Briefly, mouse livers were perfused with PBS and a single cell suspension was generated. Hepatocytes were removed via centrifugation at 40 x g two times. Non-parenchymal cells were retrieved via a 21% iodixanol gradient (20 min, 400 x g, no brake) and stimulated with PMA/Ionomycin for 4 h at 37°C. NPCs were stained with Pacific Orange to exclude dead cells and stained on the surface with fluorochrome-labelled antibodies directed against CD4 (clone RM4-5, BioLegend) and CD45.1 (clone A20, BioLegend). Cells were fixed with 4% PFA for 15 min at 4°C and washed with Saponin buffer to permeabilize the cells before intracellular staining for IFN-γ (clone XMG1.2, BioLegend) and CTLA-4 (clone UC10-4F10-11, BD). Cells were measured at an LSRII (BD).

To determine the expression of the genes encoding CXCL9 and CXCR3, RNA was isolated from whole liver tissue with the NucleoSpin RNA Kit (Macherey-Nagel) and reverse transcribed with the High Capacity cDNA Reverse Transcription Kit (Thermo Fisher). qPCR was performed with Taqman probes (Thermo Fisher) and the KAPA Probe Fast qPCR Master Mix (KAPA Biosystems). The following probes were used: Hprt (housekeeping): Mm00446968_ml; CXCL9: Mm00434946_m1, CXCR3: Mm99999054_s1 (all obtained from Thermo Fisher).

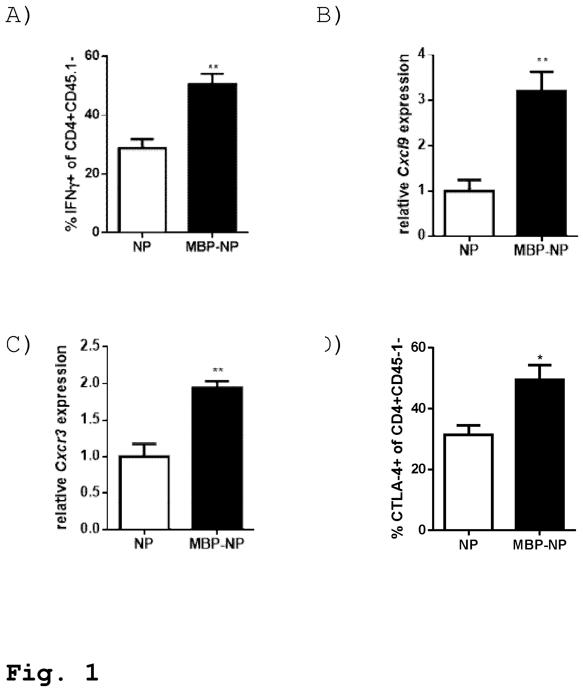

Results: It can be seen in Fig. 1A that after administration of MBP-coupled nanoparticles, MBP-specific T cells in the liver increased the production of IFN-γ as compared to administration of unloaded nanoparticles. Apart from IFN-γ, the expression of the IFN-γ-induced molecules CXCL9 (Fig. 1B) and CXCR3 (Fig. 1C) were also found to be up-regulated in the liver in response to administration of MBP-coupled nanoparticles. Moreover, the co-inhibitory molecule CTLA-4 was up-regulated on MBP-specific T cells in the liver of mice treated with MBP-coupled nanoparticles (Fig. 1D). These findings indicate that IFN-γ, CXCL9, CXCR3 and CTLA-4 can serve as biomarkers for successful hepatic tolerance induction by nanoparticle administration.

### Example 3: IFN-γ inhibition leads to loss of immune protection

To assess the relevance of IFN-γ for MBP-nanoparticle-induced immune tolerance, IFN-γ was blocked in vivo by administration of anti-IFN-γ antibody in the multiple sclerosis model of MBP-induced experimental autoimmune encephalomyelitis (EAE). Mice were immunized to MBP and injected with MBP-loaded or control nanoparticles as well as antigen-specific CD4 T cells as described above. 300 µg of anti-IFN-γ (clone XMG1.2, BioXCell) or appropriate isotype antibody was injected intraperitoneally twice a week. EAE score was monitored daily and symptoms were scored as follows: 0, no signs of disease; 1, flaccid tail; 2, partial hind-limb paralysis; 3, complete hind-limb paralysis; 4, fore- and hind-limb paralysis; 5, moribund. Treatment with anti-IFN-γ antibody is associated with atypical EAE symptoms in some mice. These are scored as follows: 0, no clinical signs; 1, slight head tilt; 2, severe head tilt; 3, slight axial rotation/staggered walking; 4, severe axial rotation/spinning; 5, moribund.

Results: It was found that by inhibiting the IFN-γ signal, EAE could be induced in animals that were otherwise immunotolerant after administration of MBP-coupled nanoparticles. This demonstrates that the upregulation of IFN-γ in autoreactive cells is crucial to the MBP tolerance of the liver.

### Example 4: Measuring of co-inhibitor expression levels in blood and spleen

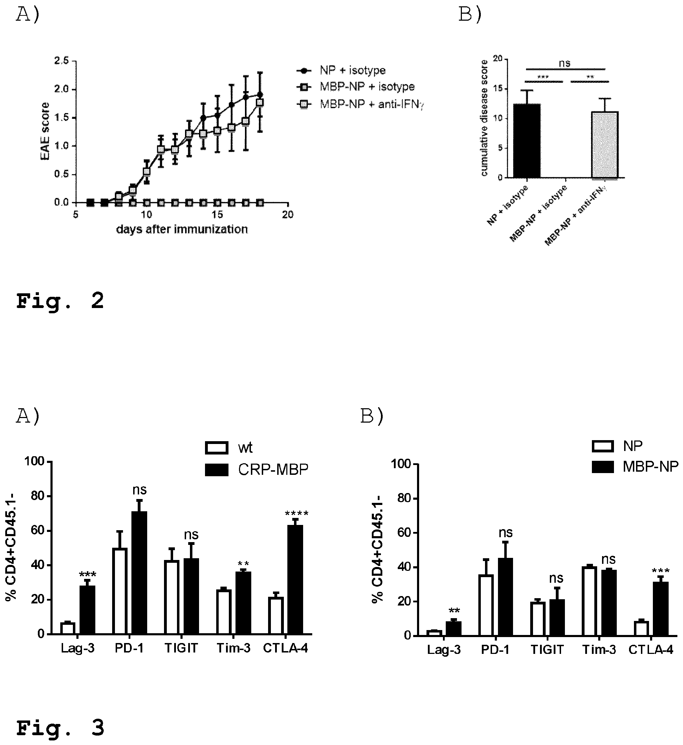

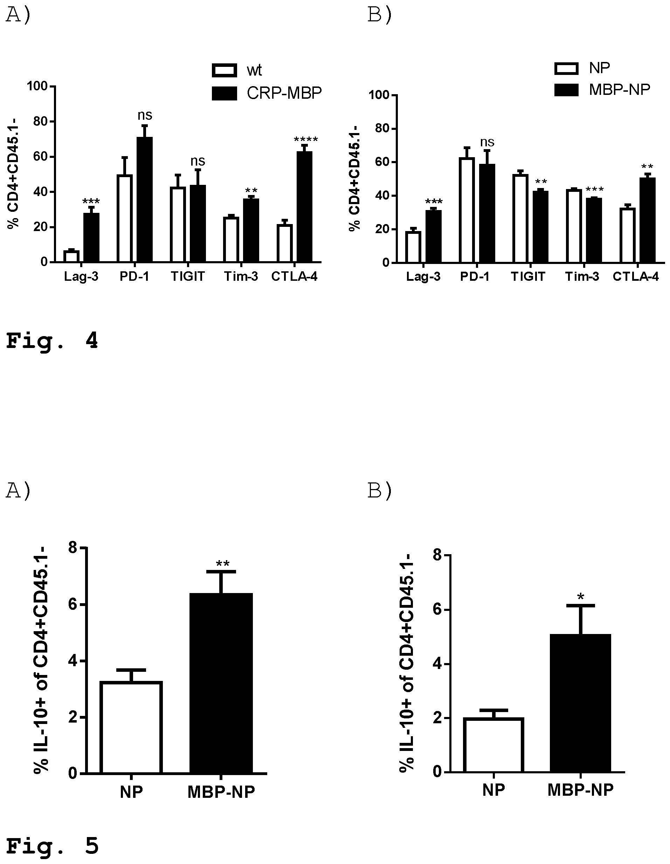

To identify tolerance biomarkers of MBP-specific T cells that have been tolerized in the liver, MBP-specific T cells were isolated from blood (Fig. 3) and spleen (Fig.4) and were re-analyzed ex vivo. These analyses were performed in two models of hepatic tolerance that was (A) induced by MBP gene transfer in CRP-MBP mice (Lüth et al. (2008), J. Clin. Invest. 118: 3403-3410), or (B) by MBP peptide-loaded nanoparticles (Carambia et al. (2015), J. Hepatol. 62:1349-56). Spleens were mechanically dissected and a single cell suspension was generated. Erythrocytes were lysed by incubation in 1 mL ACK buffer for 90s. For analysis of peripheral blood cells, 100 µl of blood was drawn from the vena cava inferior and transferred to an Eppendorf tube filled with heparin (10 IU heparin per ml blood). Erythrocyte lysis was performed by incubation in 1 ml ACK (ammonium chloride potassium) lysis buffer for 5 min. If necessary, erythrocyte lysis was repeated up to two times. Expression of co-inhibitory receptors (Lag-3, clone C9B7W, BioLegend; PD-1, clone 29F.1A12; BioLegend; TIGIT, clone 1G9, BD; Tim-3, clone RMT3-23, BioLegend; CTLA-4, clone UC10-4F10-11, BD), was analyzed via flow cytometry as described above.

Results: As can be seen in Fig. 3, the co-inhibitory molecules LAG3, PD-1 (PDCD1), TIGIT, TIM3 (HAVCR2) and CTLA-4 were found to be increased in MBP-specific T cells in the blood 7 days after MBP immunization (Fig.3). Increased expression of these co-inhibitors was also detected in MBP-specific T cells in the spleen 7 days after MBP immunization (Fig.4). These findings indicate that hepatic tolerance induction is associated with increased expression of co-inhibitory molecules which can thus serve as biomarkers indicating therapeutic efficacy.

### Example 5: Measuring of IL-10 expression levels in blood and liver

MBP-specific T cells from liver and blood were isolated as described above. Cells were stained with Pacific Orange to exclude dead cells and stained on the surface with fluorochrome-labelled antibodies directed against CD4 (clone RM4-5, BioLegend) and CD45.1 (clone A20, BioLegend). Cells were fixed with 4% PFA for 15 min at 4°C and washed with Saponin buffer to permeabilize the cells before intracellular staining for Interleukin 10 (clone JES5-16E3, BioLegend). Cells were measured at an LSRII (BD).

Results: As can be seen in Fig. 5, administration of nanoparticles loaded with MBP peptide induced Interleukin-10 production by MBP-specific T cells in the liver (A), and in the blood (B), measured on day 7 after immunization to MBP.

## Claims

1. Method for determining the effectiveness of a nanoparticle-based therapy for suppressing an immune response in a subject, said method comprising
(a) providing a test sample from a subject to whom nanoparticles have been administered to suppress an immune response;
(b) determining in said test sample the expression level of at least one nucleotide sequence selected from the group consisting of SEQ ID NOs:1-9 or the complement thereof; and
(b) comparing the expression level of the nucleotide sequence determined in the test sample to the expression level of the same nucleotide sequence determined in a control sample,
wherein an increased expression level of the at least one nucleotide sequence in the test sample relative to the control sample indicates that the nanoparticle-based therapy is effective in said subject.

2. Method according to claim 1, wherein said test sample from said subject is a blood sample.

3. Method according to claim 2, wherein the erythrocytes are removed from the blood sample before determination of the expression levels.

4. Method according to any of claims 1-3, wherein the expression level of said at least one nucleotide sequence is determined in said test sample 2-8 days after the nanoparticles have been administered to the subject.

5. Method according to any of claims 1-4, wherein the subject suffers from an autoimmune disease, an allergy or an inflammatory disease.

6. Method according to claim 5, wherein said autoimmune disease autoimmune disease is selected from the group consisting of multiple sclerosis, type I diabetes, rheumatoid arthritis, systemic lupus erythematosus, ulcerative colitis, Crohn's disease, autoimmune cardiomyopathy, autoimmune hepatitis, Graves' disease, Guillain-Barre syndrome (GBS), Hashimoto's thyroiditis, idiopathic thrombocytopenic purpura, juvenile idiopathic arthritis, myasthenia gravis, pemphigus vulgaris, psoriasis, Reiter's syndrome, scleroderma, Sjögren's syndrome, vasculitis, vitiligo, and Wegener's granulomatosis.

7. Method according to any of claims 1-6, wherein determining the expression level in step (b) is performed at a transcriptional level by measuring mRNA levels, preferably by RT-PCR or real-time RT-PCR.

8. Method according to any of claims 1-6, wherein determining the expression level in step (b) is performed at a translational level by measuring protein levels, preferably by flow cytometry.

9. Method according to any of claims 1-8, wherein the expression levels of all nucleotide sequences depicted in SEQ ID NOs:1-9 are determined in step (b).

10. Method according to any of claims 1-9, wherein the nanoparticles that have been administered to the subject comprise
(a) a micelle comprising an amphiphilic polymer rendering the nanoparticle water-soluble, and
(b) a peptide comprising the at least one T cell epitope associated with the outside of the micelle.

11. Method according to claim 10, wherein the nanoparticles are negatively charged.

12. Method according to any of claims 10-11, wherein the amphiphilic is a synthetic polymer selected from the group consisting of poly(maleic anhydride-alt-1-octadecene), poly(maleic anhydride-alt-1-tetradecene) and polyisoprene-block polyethyleneoxide block copolymer.

13. Method according to any of claims 10-12, wherein the nanoparticle comprises a solid hydrophobic core.

14. Method according to any of claims 10-13, wherein the peptide is covalently linked to the micelle.

15. Method according to any of claims 10-14, wherein the peptide is derived from the myelin basic protein.


SEQUENCE LISTING

<110>  Universitätsklinikum Hamburg Eppendorf

<120>  METHOD FOR DETERMINING THE EFFECTIVENESS OF A NANOPARTICLE-BASED
       THERAPY

<130>  P 107760

<160>  27

<170>  PatentIn version 3.5

<210>  1
<211>  1887
<212>  DNA
<213>  Homo sapiens

<400>  1
gctttctatt caagtgcctt ctgtgtgtgc acatgtgtaa tacatatctg ggatcaaagc      60

tatctatata aagtccttga ttctgtgtgg gttcaaacac atttcaaagc ttcaggatcc     120

tgaaaggttt tgctctactt cctgaagacc tgaacaccgc tcccataaag ccatggcttg     180

ccttggattt cagcggcaca aggctcagct gaacctggct accaggacct ggccctgcac     240

tctcctgttt tttcttctct tcatccctgt cttctgcaaa gcaatgcacg tggcccagcc     300

tgctgtggta ctggccagca gccgaggcat cgccagcttt gtgtgtgagt atgcatctcc     360

aggcaaagcc actgaggtcc gggtgacagt gcttcggcag gctgacagcc aggtgactga     420

agtctgtgcg gcaacctaca tgatggggaa tgagttgacc ttcctagatg attccatctg     480

cacgggcacc tccagtggaa atcaagtgaa cctcactatc caaggactga gggccatgga     540

cacgggactc tacatctgca aggtggagct catgtaccca ccgccatact acctgggcat     600

aggcaacgga acccagattt atgtaattgc taaagaaaag aagccctctt acaacagggg     660

tctatgtgaa aatgccccca acagagccag aatgtgaaaa gcaatttcag ccttatttta     720

ttcccatcaa ttgagaaacc attatgaaga agagagtcca tatttcaatt tccaagagct     780

gaggcaattc taactttttt gctatccagc tatttttatt tgtttgtgca tttgggggga     840

attcatctct ctttaatata aagttggatg cggaacccaa attacgtgta ctacaattta     900

aagcaaagga gtagaaagac agagctggga tgtttctgtc acatcagctc cactttcagt     960

gaaagcatca cttgggatta atatggggat gcagcattat gatgtgggtc aaggaattaa    1020

gttagggaat ggcacagccc aaagaaggaa aaggcaggga gcgagggaga agactatatt    1080

gtacacacct tatatttacg tatgagacgt ttatagccga aatgatcttt tcaagttaaa    1140

ttttatgcct tttatttctt aaacaaatgt atgattacat caaggcttca aaaatactca    1200

catggctatg ttttagccag tgatgctaaa ggttgtattg catatataca tatatatata    1260

tatatatata tatatatata tatatatata tatatatata tatattttaa tttgatagta    1320

ttgtgcatag agccacgtat gtttttgtgt atttgttaat ggtttgaata taaacactat 1380

atggcagtgt ctttccacct tgggtcccag ggaagttttg tggaggagct caggacacta 1440

atacaccagg tagaacacaa ggtcatttgc taactagctt ggaaactgga tgaggtcata 1500

gcagtgcttg attgcgtgga attgtgctga gttggtgttg acatgtgctt tggggctttt 1560

acaccagttc ctttcaatgg tttgcaagga agccacagct ggtggtatct gagttgactt 1620

gacagaacac tgtcttgaag acaatggctt actccaggag acccacaggt atgaccttct 1680

aggaagctcc agttcgatgg gcccaattct tacaaacatg tggttaatgc catggacaga 1740

agaaggcagc aggtggcaga atggggtgca tgaaggtttc tgaaaattaa cactgcttgt 1800

gtttttaact caatattttc catgaaaatg caacaacatg tataatattt ttaattaaat 1860

aaaaatctgt ggtggtcgtt ttccgga 1887

<210> 2
<211> 2926
<212> DNA
<213> Homo sapiens

<400> 2
acatctgctt cctgtaggcc ctctgggcag aagcatgcgc tggtgtctcc tcctgatctg 60

ggcccagggg ctgaggcagg ctcccctcgc ctcaggaatg atgacaggca caatagaaac 120

aacggggaac atttctgcag agaaaggtgg ctctatcatc ttacaatgtc acctctcctc 180

caccacggca caagtgaccc aggtcaactg ggagcagcag gaccagcttc tggccatttg 240

taatgctgac ttggggtggc acatctcccc atccttcaag gatcgagtgg ccccaggtcc 300

cggcctgggc ctcaccctcc agtcgctgac cgtgaacgat acaggggagt acttctgcat 360

ctatcacacc taccctgatg ggacgtacac tgggagaatc ttcctggagg tcctagaaag 420

ctcagtggct gagcacggtg ccaggttcca gattccattg cttggagcca tggccgcgac 480

gctggtggtc atctgcacag cagtcatcgt ggtggtcgcg ttgactagaa agaagaaagc 540

cctcagaatc cattctgtgg aaggtgacct caggagaaaa tcagctggac aggaggaatg 600

gagccccagt gctccctcac ccccaggaag ctgtgtccag gcagaagctg cacctgctgg 660

gctctgtgga gagcagcggg gagaggactg tgccgagctg catgactact tcaatgtcct 720

gagttacaga agcctgggta actgcagctt cttcacagag actggttagc aaccagaggc 780

atcttctgga agatacactt ttgtctttgc tattatagat gaatatataa gcagctgtac 840

tctccatcag tgctgcgtgt gtgtgtgtgt gtgtatgtgt gtgtgtgttc agttgagtga 900

ataaatgtca tcctcttctc catcttcatt tccttggcct tttcgttcta ttccattttg 960

cattatggca ggcctagggt gagtaacgtg gatcttgatc ataaatgcaa aattaaaaaa 1020

tatcttgacc tggttttaaa tctggcagtt tgagcagatc ctatgtctct gagagacaca 1080

ttcctcataa tggccagcat tttgggctac aaggttttgt ggttgatgat gaggatggca 1140
tgactgcaga gccatcctca tctcattttt tcacgtcatt ttcagtaact ttcactcatt 1200
caaaggcagg ttataagtaa gtcctggtag cagcctctat ggggagattt gagagtgact 1260
aaatcttggt atctgccctc aagaacttac agttaaatgg ggagacaatg ttgtcatgaa 1320
aaggtattat agtaaggaga gaaggagaca tacacaggcc ttcaggaaga gacgacagtt 1380
tggggtgagg tagttggcat aggcttatct gtgatgaagt ggcctgggag caccaagggg 1440
atgttgaggc tagtctggga ggagcaggag ttttgtctag ggaacttgta ggaaattctt 1500
ggagctgaaa gtcccacaaa gaaggccctg gcaccaaggg agtcagcaaa cttcagattt 1560
tattctctgg gcaggcattt caagtttcct tttgctgtga catactcatc cattagacag 1620
cctgatacag gcctgtagcc tcttccggcc gtgtgtgctg gggaagcccc aggaaacgca 1680
catgcccaca cagggagcca agtcgtagca tttgggcctt gatctacctt ttctgcatca 1740
atacactctt gagcctttga aaaaagaacg tttcccacta aaaagaaaat gtggattttt 1800
aaaataggga ctcttcctag ggggaaaagg ggggctggga gtgatagagg gtttaaaaaa 1860
taaacacctt caaactaact tcttcgaacc cttttattca ctccctgacg actttgtgct 1920
ggggttgggg taactgaacc gcttatttct gtttaattgc attcaggctg gatcttagaa 1980
gacttttatc cttccaccat ctctctcaga ggaatgagcg gggaggttgg atttactggt 2040
gactgatttt ctttcatggg ccaaggaact gaaagagaat gtgaagcaag gttgtgtctt 2100
gcgcatggtt aaaaataaag cattgtcctg cttcctaaga cttagactgg ggttgacaat 2160
tgttttagca acaagacaat tcaactattt ctcctaggat ttttattatt attatttttt 2220
cacttttcta ccaaatgggt tacataggaa gaatgaactg aaatctgtcc agagctccaa 2280
gtcctttgga agaaagatta gatgaacgta aaaatgttgt tgtttgctgt ggcagtttac 2340
agcatttttc ttgcaaaatt agtgcaaatc tgttggaaat agaacacaat tcacaaattg 2400
gaagtgaact aaaatgtaat gacgaaaagg gagtagtgtt ttgatttgga ggaggtgtat 2460
attcggcaga ggttggactg agagttgggt gttatttaac ataattatgg taattgggaa 2520
acatttataa acactattgg gatggtgata aaatacaaaa gggcctatag atgttagaaa 2580
tgggtcaggt tactgaaatg ggattcaatt tgaaaaaaat ttttttaaat agaactcact 2640
gaactagatt ctcctctgag aaccagagaa gaccatttca tagttggatt cctggagaca 2700
tgcgctatcc accacgtagc cactttccac atgtggccat caaccactta agatggggtt 2760
agtttaaatc aagatgtgct gttataattg gtataagcat aaaatcacac tagattctgg 2820
agatttaata tgaataataa gaatactatt tcagtagttt tggtatattg tgtgtcaaaa 2880
atgataatat tttggatgta ttgggtgaaa taaaatatta acatta 2926

<210>  3
<211>  2237
<212>  DNA
<213>  Homo sapiens

<400>  3
atttggagag ttaaaactgt gcctaacaga ggtgtcctct gacttttctt ctgcaagctc      60

catgttttca catcttccct ttgactgtgt cctgctgctg ctgctgctac tacttacaag     120

gtcctcagaa gtggaataca gagcggaggt cggtcagaat gcctatctgc cctgcttcta     180

caccccagcc gccccaggga acctcgtgcc cgtctgctgg ggcaaaggag cctgtcctgt     240

gtttgaatgt ggcaacgtgg tgctcaggac tgatgaaagg gatgtgaatt attggacatc     300

cagatactgg ctaaatgggg atttccgcaa aggagatgtg tccctgacca tagagaatgt     360

gactctagca gacagtggga tctactgctg ccggatccaa atcccaggca taatgaatga     420

tgaaaaattt aacctgaagt tggtcatcaa accagccaag gtcacccctg caccgactcg     480

gcagagagac ttcactgcag cctttccaag gatgcttacc accaggggac atggcccagc     540

agagacacag acactgggga gcctccctga tataaatcta acacaaatat ccacattggc     600

caatgagtta cgggactcta gattggccaa tgacttacgg gactctggag caaccatcag     660

aataggcatc tacatcggag cagggatctg tgctgggctg gctctggctc ttatcttcgg     720

cgctttaatt ttcaaatggt attctcatag caaagagaag atacagaatt taagcctcat     780

ctctttggcc aacctccctc cctcaggatt ggcaaatgca gtagcagagg gaattcgctc     840

agaagaaaac atctatacca ttgaagagaa cgtatatgaa gtggaggagc ccaatgagta     900

ttattgctat gtcagcagca ggcagcaacc ctcacaacct ttgggttgtc gctttgcaat     960

gccatagatc caaccacctt atttttgagc ttggtgtttt gtctttttca gaaactatga    1020

gctgtgtcac ctgactggtt ttggaggttc tgtccactgc tatggagcag agttttccca    1080

ttttcagaag ataatgactc acatgggaat tgaactggga cctgcactga acttaaacag    1140

gcatgtcatt gcctctgtat ttaagccaac agagttaccc aacccagaga ctgttaatca    1200

tggatgttag agctcaaacg ggcttttata tacactagga attcttgacg tggggtctct    1260

ggagctccag gaaattcggg cacatcatat gtccatgaaa cttcagataa actagggaaa    1320

actgggtgct gaggtgaaag cataactttt ttggcacaga aagtctaaag gggccactga    1380

ttttcaaaga gatctgtgat ccctttttgt tttttgtttt tgagatggag tcttgctctg    1440

ttgcccaggc tggagtgcaa tggcacaatc tcggctcact gcaagctccg cctcctgggt    1500

tcaagcgatt ctcctgcctc agcctcctga gtggctggga ttacaggcat gcaccaccat    1560

gcccagctaa tttgttgtat ttttagtaga gacagggttt caccatgttg gccagtgtgg    1620

tctcaaactc ctgacctcat gatttgcctg cctcggcctc ccaaagcact gggattacag    1680

gcgtgagcca ccacatccag ccagtgatcc ttaaaagatt aagagatgac tggaccaggt    1740

ctaccttgat cttgaagatt cccttggaat gttgagattt aggcttattt gagcactgcc 1800

tgcccaactg tcagtgccag tgcatagccc ttcttttgtc tcccttatga agactgccct 1860

gcagggctga gatgtggcag gagctcccag ggaaaaacga agtgcatttg attggtgtgt 1920

attggccaag ttttgcttgt tgtgtgcttg aaagaaaata tctctgacca acttctgtat 1980

tcgtggacca aactgaagct atatttttca cagaagaaga agcagtgacg gggacacaaa 2040

ttctgttgcc tggtggaaag aaggcaaagg ccttcagcaa tctatattac cagcgctgga 2100

tcctttgaca gagagtggtc cctaaactta aatttcaaga cggtataggc ttgatctgtc 2160

ttgcttattg ttgccccctg cgcctagcac aattctgaca cacaattgga acttactaaa 2220

aatttttttt tactgtt 2237

<210> 4
<211> 1976
<212> DNA
<213> Homo sapiens

<400> 4
agagaccagc agaacggcat cccagccacg acggccactt tgctctgtct gctctccgcc 60

acggccctgc tctgttccct gggacacccc cgcccccacc tcctcaggct gcctgatctg 120

cccagctttc cagctttcct ctggattccg gcctctggtc atccctcccc accctctctc 180

caaggccctc tcctggtctc ccttcttcta gaaccccttc ctccacctcc ctctctgcag 240

aacttctcct ttacccccca ccccccacca ctgccccctt tccttttctg acctcctttt 300

ggagggctca gcgctgccca gaccatagga gagatgtggg aggctcagtt cctgggcttg 360

ctgtttctgc agccgctttg ggtggctcca gtgaagcctc tccagccagg ggctgaggtc 420

ccggtggtgt gggcccagga gggggctcct gcccagctcc cctgcagccc cacaatcccc 480

ctccaggatc tcagccttct gcgaagagca ggggtcactt ggcagcatca gccagacagt 540

ggcccgcccg ctgccgcccc cggccatccc ctggcccccg gccctcaccc ggcggcgccc 600

tcctcctggg ggcccaggcc ccgccgctac acggtgctga gcgtgggtcc cggaggcctg 660

cgcagcggga ggctgcccct gcagccccgc gtccagctgg atgagcgcgg ccggcagcgc 720

ggggacttct cgctatggct gcgcccagcc cggcgcgcgg acgccggcga gtaccgcgcc 780

gcggtgcacc tcagggaccg cgccctctcc tgccgcctcc gtctgcgcct gggccaggcc 840

tcgatgactg ccagcccccc aggatctctc agagcctccg actgggtcat tttgaactgc 900

tccttcagcc gccctgaccg cccagcctct gtgcattggt tccggaaccg gggccagggc 960

cgagtccctg tccgggagtc cccccatcac cacttagcgg aaagcttcct cttcctgccc 1020

caagtcagcc ccatggactc tgggccctgg ggctgcatcc tcacctacag agatggcttc 1080

aacgtctcca tcatgtataa cctcactgtt ctgggtctgg agcccccaac tcccttgaca 1140

gtgtacgctg gagcaggttc cagggtgggg ctgccctgcc gcctgcctgc tggtgtgggg 1200

acccggtctt tcctcactgc caagtggact cctcctgggg gaggccctga cctcctggtg 1260

actggagaca atggcgactt tacccttcga ctagaggatg tgagccaggc ccaggctggg 1320

acctacacct gccatatcca tctgcaggaa cagcagctca atgccactgt cacattggca 1380

atcatcacag tgactcccaa atcctttggg tcacctggat ccctggggaa gctgctttgt 1440

gaggtgactc cagtatctgg acaagaacgc tttgtgtgga gctctctgga caccccatcc 1500

cagaggagtt tctcaggacc ttggctggag gcacaggagg cccagctcct ttcccagcct 1560

tggcaatgcc agctgtacca gggggagagg cttcttggag cagcagtgta cttcacagag 1620

ctgtctagcc caggtgccca acgctctggg agagccccag gtgccctccc agcaggccac 1680

ctcctgctgt ttctcatcct tggtgtcctt tctctgctcc ttttggtgac tggagccttt 1740

ggctttcacc tttggagaag acagtggcga ccaagacgat tttctgcctt agagcaaggg 1800

attcaccctc cgcaggctca gagcaagata gaggagctgg agcaagaacc ggagccggag 1860

ccggagccgg aaccggagcc cgagcccgag cccgagccgg agcagctctg acctggagct 1920

gaggcagcca gcagatctca gcagcccagt ccaaataaac tccctgtcag cagcaa 1976

<210> 5
<211> 2097
<212> DNA
<213> Homo sapiens

<400> 5
gctcacctcc gcctgagcag tggagaaggc ggcactctgg tggggctgct ccaggcatgc 60

agatcccaca ggcgccctgg ccagtcgtct gggcggtgct acaactgggc tggcggccag 120

gatggttctt agactcccca gacaggccct ggaacccccc caccttctcc ccagccctgc 180

tcgtggtgac cgaaggggac aacgccacct tcacctgcag cttctccaac acatcggaga 240

gcttcgtgct aaactggtac cgcatgagcc ccagcaacca gacggacaag ctggccgcct 300

tccccgagga ccgcagccag cccggccagg actgccgctt ccgtgtcaca caactgccca 360

acgggcgtga cttccacatg agcgtggtca gggcccggcg caatgacagc ggcacctacc 420

tctgtggggc catctccctg gcccccaagg cgcagatcaa agagagcctg cgggcagagc 480

tcagggtgac agagagaagg gcagaagtgc ccacagccca ccccagcccc tcacccaggc 540

cagccggcca gttccaaacc ctggtggttg gtgtcgtggg cggcctgctg ggcagcctgg 600

tgctgctagt ctgggtcctg gccgtcatct gctcccgggc cgcacgaggg acaataggag 660

ccaggcgcac cggccagccc ctgaaggagg acccctcagc cgtgcctgtg ttctctgtgg 720

actatgggga gctggatttc cagtggcgag agaagacccc ggagcccccc gtgccctgtg 780

tccctgagca gacggagtat gccaccattg tctttcctag cggaatgggc acctcatccc 840

ccgcccgcag gggctcagct gacggccctc ggagtgccca gccactgagg cctgaggatg 900

gacactgctc ttggcccctc tgaccggctt ccttggccac cagtgttctg cagaccctcc 960

accatgagcc cgggtcagcg catttcctca ggagaagcag gcagggtgca ggccattgca 1020

ggccgtccag gggctgagct gcctgggggc gaccggggct ccagcctgca cctgcaccag 1080

gcacagcccc accacaggac tcatgtctca atgcccacag tgagcccagg cagcaggtgt 1140

caccgtcccc tacagggagg gccagatgca gtcactgctt caggtcctgc cagcacagag 1200

ctgcctgcgt ccagctccct gaatctctgc tgctgctgct gctgctgctg ctgctgcctg 1260

cggcccgggg ctgaaggcgc cgtggccctg cctgacgccc cggagcctcc tgcctgaact 1320

tgggggctgg ttggagatgg ccttggagca gccaaggtgc ccctggcagt ggcatcccga 1380

aacgccctgg acgcagggcc caagactggg cacaggagtg ggaggtacat ggggctgggg 1440

actccccagg agttatctgc tccctgcagg cctagagaag tttcagggaa ggtcagaaga 1500

gctcctggct gtggtgggca gggcaggaaa cccctccacc tttacacatg cccaggcagc 1560

acctcaggcc ctttgtgggg cagggaagct gaggcagtaa gcgggcaggc agagctggag 1620

gcctttcagg cccagccagc actctggcct cctgccgccg cattccaccc cagcccctca 1680

caccactcgg gagagggaca tcctacggtc ccaaggtcag gagggcaggg ctggggttga 1740

ctcaggcccc tcccagctgt ggccacctgg gtgttgggag ggcagaagtg caggcaccta 1800

gggcccccca tgtgcccacc ctgggagctc tccttggaac ccattcctga aattatttaa 1860

aggggttggc cgggctccca ccagggcctg ggtgggaagg tacaggcgtt cccccggggc 1920

ctagtacccc cgccgtggcc tatccactcc tcacatccac acactgcacc cccactcctg 1980

gggcagggcc accagcatcc aggcggccag caggcacctg agtggctggg acaagggatc 2040

ccccttccct gtggttctat tatattataa ttataattaa atatgagagc atgctaa 2097

<210> 6
<211> 1211
<212> DNA
<213> Homo sapiens

<400> 6
acattgttct gatcatctga agatcagcta ttagaagaga aagatcagtt aagtcctttg 60

gacctgatca gcttgataca agaactactg atttcaactt ctttggctta attctctcgg 120

aaacgatgaa atatacaagt tatatcttgg cttttcagct ctgcatcgtt ttgggttctc 180

ttggctgtta ctgccaggac ccatatgtaa aagaagcaga aaaccttaag aaatatttta 240

atgcaggtca ttcagatgta gcggataatg gaactctttt cttaggcatt ttgaagaatt 300

ggaaagagga gagtgacaga aaaataatgc agagccaaat tgtctccttt tacttcaaac 360

tttttaaaaa ctttaaagat gaccagagca tccaaaagag tgtggagacc atcaaggaag 420

acatgaatgt caagtttttc aatagcaaca aaaagaaacg agatgacttc gaaaagctga 480

ctaattattc ggtaactgac ttgaatgtcc aacgcaaagc aatacatgaa ctcatccaag 540

tgatggctga actgtcgcca gcagctaaaa cagggaagcg aaaaaggagt cagatgctgt 600

ttcgaggtcg aagagcatcc cagtaatggt tgtcctgcct gcaatatttg aattttaaat 660

ctaaatctat ttattaatat ttaacattat ttatatgggg aatatatttt tagactcatc 720

aatcaaataa gtatttataa tagcaacttt tgtgtaatga aaatgaatat ctattaatat 780

atgtattatt tataattcct atatcctgtg actgtctcac ttaatccttt gttttctgac 840

taattaggca aggctatgtg attacaaggc tttatctcag gggccaacta ggcagccaac 900

ctaagcaaga tcccatgggt tgtgtgttta tttcacttga tgatacaatg aacacttata 960

agtgaagtga tactatccag ttactgccgg tttgaaaata tgcctgcaat ctgagccagt 1020

gctttaatgg catgtcagac agaacttgaa tgtgtcaggt gaccctgatg aaaacatagc 1080

atctcaggag atttcatgcc tggtgcttcc aaatattgtt gacaactgtg actgtaccca 1140

aatggaaagt aactcatttg ttaaaattat caatatctaa tatatatgaa taaagtgtaa 1200

gttcacaact a 1211

<210> 7
<211> 2761
<212> DNA
<213> Homo sapiens

<400> 7
aaagaatttc tcaggctcaa aatccaatac aggagtgact tggaactcca ttctatcact 60

atgaagaaaa gtggtgttct tttcctcttg ggcatcatct tgctggttct gattggagtg 120

caaggaaccc cagtagtgag aaagggtcgc tgttcctgca tcagcaccaa ccaagggact 180

atccacctac aatccttgaa agaccttaaa caatttgccc caagcccttc ctgcgagaaa 240

attgaaatca ttgctacact gaagaatgga gttcaaacat gtctaaaccc agattcagca 300

gatgtgaagg aactgattaa aaagtgggag aaacaggtca gccaaaagaa aaagcaaaag 360

aatgggaaaa aacatcaaaa aaagaaagtt ctgaaagttc gaaaatctca acgttctcgt 420

caaaagaaga ctacataaga gaccacttca ccaataagta ttctgtgtta aaaatgttct 480

attttaatta taccgctatc attccaaagg aggatggcat ataatacaaa ggcttattaa 540

tttgactaga aaatttaaaa cattactctg aaattgtaac taaagttaga aagttgattt 600

taagaatcca aacgttaaga attgttaaag gctatgattg tctttgttct tctaccaccc 660

accagttgaa tttcatcatg cttaaggcca tgattttagc aatacccatg tctacacaga 720

tgttcaccca accacatccc actcacaaca gctgcctgga agagcagccc taggcttcca 780

cgtactgcag cctccagaga gtatctgagg cacatgtcag caagtcctaa gcctgttagc 840

atgctggtga gccaagcagt ttgaaattga gctggacctc accaagctgc tgtggccatc 900

aacctctgta tttgaatcag cctacaggcc tcacacacaa tgtgtctgag agattcatgc 960

tgattgttat tgggtatcac cactggagat caccagtgtg tggctttcag agcctccttt 1020

ctggctttgg aagccatgtg attccatctt gcccgctcag gctgaccact ttatttcttt 1080

ttgttcccct ttgcttcatt caagtcagct cttctccatc ctaccacaat gcagtgcctt 1140

tcttctctcc agtgcacctg tcatatgctc tgatttatct gagtcaactc ctttctcatc 1200

ttgtccccaa caccccacag aagtgctttc ttctcccaat tcatcctcac tcagtccagc 1260

ttagttcaag tcctgcctct taaataaacc tttttggaca cacaaattat cttaaaactc 1320

ctgtttcact tggttcagta ccacatgggt gaacactcaa tggttaacta attcttgggt 1380

gtttatccta tctctccaac cagattgtca gctccttgag ggcaagagcc acagtatatt 1440

tccctgtttc ttccacagtg cctaataata ctgtggaact aggttttaat aattttttaa 1500

ttgatgttgt tatgggcagg atggcaacca gaccattgtc tcagagcagg tgctggctct 1560

ttcctggcta ctccatgttg gctagcctct ggtaacctct tacttattat cttcaggaca 1620

ctcactacag ggaccaggga tgatgcaaca tccttgtctt tttatgacag gatgtttgct 1680

cagcttctcc aacaataaga agcacgtggt aaaacacttg cggatattct ggactgtttt 1740

taaaaaatat acagtttacc gaaaatcata taatcttaca atgaaaagga ctttatagat 1800

cagccagtga ccaacctttt cccaaccata caaaaattcc ttttcccgaa ggaaaagggc 1860

tttctcaata agcctcagct ttctaagatc taacaagata gccaccgaga tccttatcga 1920

aactcatttt aggcaaatat gagttttatt gtccgtttac ttgtttcaga gtttgtattg 1980

tgattatcaa ttaccacacc atctcccatg aagaaaggga acggtgaagt actaagcgct 2040

agaggaagca gccaagtcgg ttagtggaag catgattggt gcccagttag cctctgcagg 2100

atgtggaaac ctccttccag gggaggttca gtgaattgtg taggagaggt tgtctgtggc 2160

cagaatttaa acctatactc actttcccaa attgaatcac tgctcacact gctgatgatt 2220

tagagtgctg tccggtggag atcccacccg aacgtcttat ctaatcatga aactccctag 2280

ttccttcatg taacttccct gaaaaatcta agtgtttcat aaatttgaga gtctgtgacc 2340

cacttacctt gcatctcaca ggtagacagt atataactaa caaccaaaga ctacatattg 2400

tcactgacac acacgttata atcatttatc atatatatac atacatgcat acactctcaa 2460

agcaaataat ttttcacttc aaaacagtat tgacttgtat accttgtaat ttgaaatatt 2520

ttctttgtta aaatagaatg gtatcaataa atagaccatt aatcagaaaa cagatcttga 2580

ttttttttct cttgaatgta cccttcaact gttgaatgtt taatagtaaa tcttatatgt 2640

ccttatttac tttttagctt tctctcaaat aaagtgtaac actagttgag ataacacatg 2700

aaagctcttt aaagggtcga tcgggaacag gaaaaaaaac ctatggaaaa tatgacaaca 2760

c 2761

<210> 8
<211> 1859
<212> DNA
<213> Homo sapiens

<400> 8
acaagcacca aagcagaggg gcaggcagca caccacccag cagccagagc accagcccag 60

ccatggtcct tgaggggtcc ctgggccgat gggatcacgc agaagaatgc gagagaagca 120

gcctttgaga agggaagtca ctatcccaga gcccaggctg agcggatgga gttgaggaag 180

tacggccctg gaagactggc ggggacagtt ataggaggag ctgctcagag taaatcacag 240

actaaatcag actcaatcac aaaagagttc ctgccaggcc tttacacagc cccttcctcc 300

ccgttcccgc cctcacaggt gagtgaccac caagtgctaa atgacgccga ggttgccgcc 360

ctcctggaga acttcagctc ttcctatgac tatggagaaa acgagagtga ctcgtgctgt 420

acctccccgc cctgcccaca ggacttcagc ctgaacttcg accgggcctt cctgccagcc 480

ctctacagcc tcctctttct gctggggctg ctgggcaacg gcgcggtggc agccgtgctg 540

ctgagccggc ggacagccct gagcagcacc gacaccttcc tgctccacct agctgtagca 600

gacacgctgc tggtgctgac actgccgctc tgggcagtgg acgctgccgt ccagtgggtc 660

tttggctctg gcctctgcaa agtggcaggt gccctcttca acatcaactt ctacgcagga 720

gccctcctgc tggcctgcat cagctttgac cgctacctga acatagttca tgccacccag 780

ctctaccgcc gggggccccc ggcccgcgtg accctcacct gcctggctgt ctgggggctc 840

tgcctgcttt tcgccctccc agacttcatc ttcctgtcgg cccaccacga cgagcgcctc 900

aacgccaccc actgccaata caacttccca caggtgggcc gcacggctct gcgggtgctg 960

cagctggtgg ctggctttct gctgcccctg ctggtcatgg cctactgcta tgcccacatc 1020

ctggccgtgc tgctggtttc caggggccag cggcgcctgc gggccatgcg gctggtggtg 1080

gtggtcgtgg tggcctttgc cctctgctgg accccctatc acctggtggt gctggtggac 1140

atcctcatgg acctgggcgc tttggcccgc aactgtggcc gagaaagcag ggtagacgtg 1200

gccaagtcgg tcacctcagg cctgggctac atgcactgct gcctcaaccc gctgctctat 1260

gcctttgtag gggtcaagtt ccgggagcgg atgtggatgc tgctcttgcg cctgggctgc 1320

cccaaccaga gagggctcca gaggcagcca tcgtcttccc gccgggattc atcctggtct 1380

gagacctcag aggcctccta ctcgggcttg tgaggccgga atccgggctc ccctttcgcc 1440

cacagtctga cttccccgca ttccaggctc ctccctccct ctgccggctc tggctctccc 1500

caatatcctc gctcccggga ctcactggca gccccagcac caccaggtct cccgggaagc 1560

caccctccca gctctgagga ctgcaccatt gctgctcctt agctgccaag ccccatcctg 1620

ccgcccgagg tggctgcctg gagccccact gcccttctca tttggaaact aaaacttcat 1680

cttccccaag tgcggggagt acaaggcatg gcgtagaggg tgctgcccca tgaagccaca 1740

gcccaggcct ccagctcagc agtgactgtg gccatggtcc ccaagacctc tatatttgct 1800

cttttatttt tatgtctaaa atcctgctta aaacttttca ataaacaaga tcgtcagga 1859

<210> 9
<211> 1630
<212> DNA
<213> Homo sapiens

<400> 9
acacatcagg ggcttgctct tgcaaaacca aaccacaaga cagacttgca aaagaaggca 60

tgcacagctc agcactgctc tgttgcctgg tcctcctgac tggggtgagg gccagcccag 120

gccagggcac ccagtctgag aacagctgca cccacttccc aggcaacctg cctaacatgc 180

ttcgagatct ccgagatgcc ttcagcagag tgaagacttt ctttcaaatg aaggatcagc 240

tggacaactt gttgttaaag gagtccttgc tggaggactt taagggttac ctgggttgcc 300

aagccttgtc tgagatgatc cagttttacc tggaggaggt gatgccccaa gctgagaacc 360

aagacccaga catcaaggcg catgtgaact ccctggggga gaacctgaag accctcaggc 420

tgaggctacg gcgctgtcat cgatttcttc cctgtgaaaa caagagcaag gccgtggagc 480

aggtgaagaa tgcctttaat aagctccaag agaaaggcat ctacaaagcc atgagtgagt 540

ttgacatctt catcaactac atagaagcct acatgacaat gaagatacga aactgagaca 600

tcagggtggc gactctatag actctaggac ataaattaga ggtctccaaa atcggatctg 660

gggctctggg atagctgacc cagccccttg agaaacctta ttgtacctct cttatagaat 720

atttattacc tctgatacct caacccccat ttctatttat ttactgagct tctctgtgaa 780

cgatttagaa agaagcccaa tattataatt tttttcaata tttattattt tcacctgttt 840

ttaagctgtt tccatagggt gacacactat ggtatttgag tgttttaaga taaattataa 900

gttacataag ggaggaaaaa aaatgttctt tggggagcca acagaagctt ccattccaag 960

cctgaccacg ctttctagct gttgagctgt tttccctgac ctccctctaa tttatcttgt 1020

ctctgggctt ggggcttcct aactgctaca aatactctta ggaagagaaa ccagggagcc 1080

cctttgatga ttaattcacc ttccagtgtc tcggagggat tcccctaacc tcattcccca 1140

accacttcat tcttgaaagc tgtggccagc ttgttattta taacaaccta aatttggttc 1200

taggccgggc gcggtggctc acgcctgtaa tcccagcact ttgggaggct gaggcgggtg 1260

gatcacttga ggtcaggagt tcctaaccag cctggtcaac atggtgaaac cccgtctcta 1320

ctaaaaatac aaaaattagc cgggcatggt ggcgcgcacc tgtaatccca gctacttggg 1380

aggctgaggc aagagaattg cttgaaccca ggagatggaa gttgcagtga gctgatatca 1440

tgcccctgta ctccagcctg ggtgacagag caagactctg tctcaaaaaa taaaaataaa 1500

aataaatttg gttctaatag aactcagttt taactagaat ttattcaatt cctctgggaa 1560

tgttacattg tttgtctgtc ttcatagcag attttaattt tgaataaata aatgtatctt 1620

attcacatca 1630

<210> 10
<211> 174
<212> PRT
<213> Homo sapiens

<400> 10

Met Ala Cys Leu Gly Phe Gln Arg His Lys Ala Gln Leu Asn Leu Ala
1               5                   10                  15

Thr Arg Thr Trp Pro Cys Thr Leu Leu Phe Phe Leu Leu Phe Ile Pro
            20                  25                  30

Val Phe Cys Lys Ala Met His Val Ala Gln Pro Ala Val Val Leu Ala
        35                  40                  45

Ser Ser Arg Gly Ile Ala Ser Phe Val Cys Glu Tyr Ala Ser Pro Gly
    50                  55                  60

Lys Ala Thr Glu Val Arg Val Thr Val Leu Arg Gln Ala Asp Ser Gln
65                  70                  75                  80

Val Thr Glu Val Cys Ala Ala Thr Tyr Met Met Gly Asn Glu Leu Thr
                85                  90                  95

Phe Leu Asp Asp Ser Ile Cys Thr Gly Thr Ser Ser Gly Asn Gln Val
            100                 105                 110

Asn Leu Thr Ile Gln Gly Leu Arg Ala Met Asp Thr Gly Leu Tyr Ile
        115                 120                 125

Cys Lys Val Glu Leu Met Tyr Pro Pro Pro Tyr Tyr Leu Gly Ile Gly
    130                 135                 140

Asn Gly Thr Gln Ile Tyr Val Ile Ala Lys Glu Lys Lys Pro Ser Tyr
145                 150                 155                 160

Asn Arg Gly Leu Cys Glu Asn Ala Pro Asn Arg Ala Arg Met
                165                 170

<210> 11

<211>  244
<212>  PRT
<213>  Homo sapiens

<400>  11

Met Arg Trp Cys Leu Leu Leu Ile Trp Ala Gln Gly Leu Arg Gln Ala
1               5                   10                  15

Pro Leu Ala Ser Gly Met Met Thr Gly Thr Ile Glu Thr Thr Gly Asn
            20                  25                  30

Ile Ser Ala Glu Lys Gly Gly Ser Ile Ile Leu Gln Cys His Leu Ser
        35                  40                  45

Ser Thr Thr Ala Gln Val Thr Gln Val Asn Trp Glu Gln Gln Asp Gln
    50                  55                  60

Leu Leu Ala Ile Cys Asn Ala Asp Leu Gly Trp His Ile Ser Pro Ser
65                  70                  75                  80

Phe Lys Asp Arg Val Ala Pro Gly Pro Gly Leu Gly Leu Thr Leu Gln
                85                  90                  95

Ser Leu Thr Val Asn Asp Thr Gly Glu Tyr Phe Cys Ile Tyr His Thr
            100                 105                 110

Tyr Pro Asp Gly Thr Tyr Thr Gly Arg Ile Phe Leu Glu Val Leu Glu
        115                 120                 125

Ser Ser Val Ala Glu His Gly Ala Arg Phe Gln Ile Pro Leu Leu Gly
    130                 135                 140

Ala Met Ala Ala Thr Leu Val Val Ile Cys Thr Ala Val Ile Val Val
145                 150                 155                 160

Val Ala Leu Thr Arg Lys Lys Lys Ala Leu Arg Ile His Ser Val Glu
                165                 170                 175

Gly Asp Leu Arg Arg Lys Ser Ala Gly Gln Glu Glu Trp Ser Pro Ser
            180                 185                 190

Ala Pro Ser Pro Pro Gly Ser Cys Val Gln Ala Glu Ala Ala Pro Ala
        195                 200                 205

Gly Leu Cys Gly Glu Gln Arg Gly Glu Asp Cys Ala Glu Leu His Asp
    210                 215                 220

Tyr Phe Asn Val Leu Ser Tyr Arg Ser Leu Gly Asn Cys Ser Phe Phe

225                 230                 235                 240

Thr Glu Thr Gly

<210>  12
<211>  301
<212>  PRT
<213>  Homo sapiens

<400>  12

Met Phe Ser His Leu Pro Phe Asp Cys Val Leu Leu Leu Leu Leu Leu
1               5                   10                  15

Leu Leu Thr Arg Ser Ser Glu Val Glu Tyr Arg Ala Glu Val Gly Gln
            20                  25                  30

Asn Ala Tyr Leu Pro Cys Phe Tyr Thr Pro Ala Ala Pro Gly Asn Leu
        35                  40                  45

Val Pro Val Cys Trp Gly Lys Gly Ala Cys Pro Val Phe Glu Cys Gly
    50                  55                  60

Asn Val Val Leu Arg Thr Asp Glu Arg Asp Val Asn Tyr Trp Thr Ser
65                  70                  75                  80

Arg Tyr Trp Leu Asn Gly Asp Phe Arg Lys Gly Asp Val Ser Leu Thr
                85                  90                  95

Ile Glu Asn Val Thr Leu Ala Asp Ser Gly Ile Tyr Cys Cys Arg Ile
            100                 105                 110

Gln Ile Pro Gly Ile Met Asn Asp Glu Lys Phe Asn Leu Lys Leu Val
        115                 120                 125

Ile Lys Pro Ala Lys Val Thr Pro Ala Pro Thr Arg Gln Arg Asp Phe
    130                 135                 140

Thr Ala Ala Phe Pro Arg Met Leu Thr Thr Arg Gly His Gly Pro Ala
145                 150                 155                 160

Glu Thr Gln Thr Leu Gly Ser Leu Pro Asp Ile Asn Leu Thr Gln Ile
                165                 170                 175

Ser Thr Leu Ala Asn Glu Leu Arg Asp Ser Arg Leu Ala Asn Asp Leu
            180                 185                 190

Arg Asp Ser Gly Ala Thr Ile Arg Ile Gly Ile Tyr Ile Gly Ala Gly

195                 200                 205

Ile Cys Ala Gly Leu Ala Leu Ala Leu Ile Phe Gly Ala Leu Ile Phe
    210                 215                 220

Lys Trp Tyr Ser His Ser Lys Glu Lys Ile Gln Asn Leu Ser Leu Ile
225                 230                 235                 240

Ser Leu Ala Asn Leu Pro Pro Ser Gly Leu Ala Asn Ala Val Ala Glu
                245                 250                 255

Gly Ile Arg Ser Glu Glu Asn Ile Tyr Thr Ile Glu Glu Asn Val Tyr
            260                 265                 270

Glu Val Glu Glu Pro Asn Glu Tyr Tyr Cys Tyr Val Ser Ser Arg Gln
        275                 280                 285

Gln Pro Ser Gln Pro Leu Gly Cys Arg Phe Ala Met Pro
    290                 295                 300

<210>  13
<211>  525
<212>  PRT
<213>  Homo sapiens

<400>  13

Met Trp Glu Ala Gln Phe Leu Gly Leu Leu Phe Leu Gln Pro Leu Trp
1               5                   10                  15

Val Ala Pro Val Lys Pro Leu Gln Pro Gly Ala Glu Val Pro Val Val
            20                  25                  30

Trp Ala Gln Glu Gly Ala Pro Ala Gln Leu Pro Cys Ser Pro Thr Ile
        35                  40                  45

Pro Leu Gln Asp Leu Ser Leu Leu Arg Arg Ala Gly Val Thr Trp Gln
    50                  55                  60

His Gln Pro Asp Ser Gly Pro Pro Ala Ala Ala Pro Gly His Pro Leu
65                  70                  75                  80

Ala Pro Gly Pro His Pro Ala Ala Pro Ser Ser Trp Gly Pro Arg Pro
                85                  90                  95

Arg Arg Tyr Thr Val Leu Ser Val Gly Pro Gly Gly Leu Arg Ser Gly
            100                 105                 110

Arg Leu Pro Leu Gln Pro Arg Val Gln Leu Asp Glu Arg Gly Arg Gln

115                 120                 125

Arg Gly Asp Phe Ser Leu Trp Leu Arg Pro Ala Arg Arg Ala Asp Ala
    130                 135                 140

Gly Glu Tyr Arg Ala Ala Val His Leu Arg Asp Arg Ala Leu Ser Cys
145                 150                 155                 160

Arg Leu Arg Leu Arg Leu Gly Gln Ala Ser Met Thr Ala Ser Pro Pro
                165                 170                 175

Gly Ser Leu Arg Ala Ser Asp Trp Val Ile Leu Asn Cys Ser Phe Ser
            180                 185                 190

Arg Pro Asp Arg Pro Ala Ser Val His Trp Phe Arg Asn Arg Gly Gln
        195                 200                 205

Gly Arg Val Pro Val Arg Glu Ser Pro His His His Leu Ala Glu Ser
    210                 215                 220

Phe Leu Phe Leu Pro Gln Val Ser Pro Met Asp Ser Gly Pro Trp Gly
225                 230                 235                 240

Cys Ile Leu Thr Tyr Arg Asp Gly Phe Asn Val Ser Ile Met Tyr Asn
                245                 250                 255

Leu Thr Val Leu Gly Leu Glu Pro Pro Thr Pro Leu Thr Val Tyr Ala
            260                 265                 270

Gly Ala Gly Ser Arg Val Gly Leu Pro Cys Arg Leu Pro Ala Gly Val
        275                 280                 285

Gly Thr Arg Ser Phe Leu Thr Ala Lys Trp Thr Pro Pro Gly Gly Gly
    290                 295                 300

Pro Asp Leu Leu Val Thr Gly Asp Asn Gly Asp Phe Thr Leu Arg Leu
305                 310                 315                 320

Glu Asp Val Ser Gln Ala Gln Ala Gly Thr Tyr Thr Cys His Ile His
                325                 330                 335

Leu Gln Glu Gln Gln Leu Asn Ala Thr Val Thr Leu Ala Ile Ile Thr
            340                 345                 350

Val Thr Pro Lys Ser Phe Gly Ser Pro Gly Ser Leu Gly Lys Leu Leu
        355                 360                 365

Cys Glu Val Thr Pro Val Ser Gly Gln Glu Arg Phe Val Trp Ser Ser
    370                 375                 380

Leu Asp Thr Pro Ser Gln Arg Ser Phe Ser Gly Pro Trp Leu Glu Ala
385                 390                 395                 400

Gln Glu Ala Gln Leu Leu Ser Gln Pro Trp Gln Cys Gln Leu Tyr Gln
                405                 410                 415

Gly Glu Arg Leu Leu Gly Ala Ala Val Tyr Phe Thr Glu Leu Ser Ser
            420                 425                 430

Pro Gly Ala Gln Arg Ser Gly Arg Ala Pro Gly Ala Leu Pro Ala Gly
        435                 440                 445

His Leu Leu Leu Phe Leu Ile Leu Gly Val Leu Ser Leu Leu Leu Leu
    450                 455                 460

Val Thr Gly Ala Phe Gly Phe His Leu Trp Arg Arg Gln Trp Arg Pro
465                 470                 475                 480

Arg Arg Phe Ser Ala Leu Glu Gln Gly Ile His Pro Pro Gln Ala Gln
                485                 490                 495

Ser Lys Ile Glu Glu Leu Glu Gln Glu Pro Glu Pro Glu Pro Glu Pro
            500                 505                 510

Glu Pro Glu Pro Glu Pro Glu Pro Glu Pro Glu Gln Leu
        515                 520                 525

<210>  14
<211>  288
<212>  PRT
<213>  Homo sapiens

<400>  14

Met Gln Ile Pro Gln Ala Pro Trp Pro Val Val Trp Ala Val Leu Gln
1               5                   10                  15

Leu Gly Trp Arg Pro Gly Trp Phe Leu Asp Ser Pro Asp Arg Pro Trp
            20                  25                  30

Asn Pro Pro Thr Phe Ser Pro Ala Leu Leu Val Val Thr Glu Gly Asp
        35                  40                  45

Asn Ala Thr Phe Thr Cys Ser Phe Ser Asn Thr Ser Glu Ser Phe Val
    50                  55                  60

Leu Asn Trp Tyr Arg Met Ser Pro Ser Asn Gln Thr Asp Lys Leu Ala
65                  70                  75                  80

Ala Phe Pro Glu Asp Arg Ser Gln Pro Gly Gln Asp Cys Arg Phe Arg
                85                  90                  95

Val Thr Gln Leu Pro Asn Gly Arg Asp Phe His Met Ser Val Val Arg
            100                 105                 110

Ala Arg Arg Asn Asp Ser Gly Thr Tyr Leu Cys Gly Ala Ile Ser Leu
        115                 120                 125

Ala Pro Lys Ala Gln Ile Lys Glu Ser Leu Arg Ala Glu Leu Arg Val
    130                 135                 140

Thr Glu Arg Arg Ala Glu Val Pro Thr Ala His Pro Ser Pro Ser Pro
145                 150                 155                 160

Arg Pro Ala Gly Gln Phe Gln Thr Leu Val Val Gly Val Val Gly Gly
                165                 170                 175

Leu Leu Gly Ser Leu Val Leu Leu Val Trp Val Leu Ala Val Ile Cys
            180                 185                 190

Ser Arg Ala Ala Arg Gly Thr Ile Gly Ala Arg Arg Thr Gly Gln Pro
        195                 200                 205

Leu Lys Glu Asp Pro Ser Ala Val Pro Val Phe Ser Val Asp Tyr Gly
    210                 215                 220

Glu Leu Asp Phe Gln Trp Arg Glu Lys Thr Pro Glu Pro Pro Val Pro
225                 230                 235                 240

Cys Val Pro Glu Gln Thr Glu Tyr Ala Thr Ile Val Phe Pro Ser Gly
                245                 250                 255

Met Gly Thr Ser Ser Pro Ala Arg Arg Gly Ser Ala Asp Gly Pro Arg
            260                 265                 270

Ser Ala Gln Pro Leu Arg Pro Glu Asp Gly His Cys Ser Trp Pro Leu
        275                 280                 285

<210>  15
<211>  166
<212>  PRT
<213>  Homo sapiens

<400>  15

Met Lys Tyr Thr Ser Tyr Ile Leu Ala Phe Gln Leu Cys Ile Val Leu
1               5                   10                  15

Gly Ser Leu Gly Cys Tyr Cys Gln Asp Pro Tyr Val Lys Glu Ala Glu
            20                  25                  30

Asn Leu Lys Lys Tyr Phe Asn Ala Gly His Ser Asp Val Ala Asp Asn
        35                  40                  45

Gly Thr Leu Phe Leu Gly Ile Leu Lys Asn Trp Lys Glu Glu Ser Asp
    50                  55                  60

Arg Lys Ile Met Gln Ser Gln Ile Val Ser Phe Tyr Phe Lys Leu Phe
65                  70                  75                  80

Lys Asn Phe Lys Asp Asp Gln Ser Ile Gln Lys Ser Val Glu Thr Ile
                85                  90                  95

Lys Glu Asp Met Asn Val Lys Phe Phe Asn Ser Asn Lys Lys Lys Arg
            100                 105                 110

Asp Asp Phe Glu Lys Leu Thr Asn Tyr Ser Val Thr Asp Leu Asn Val
        115                 120                 125

Gln Arg Lys Ala Ile His Glu Leu Ile Gln Val Met Ala Glu Leu Ser
    130                 135                 140

Pro Ala Ala Lys Thr Gly Lys Arg Lys Arg Ser Gln Met Leu Phe Arg
145                 150                 155                 160

Gly Arg Arg Ala Ser Gln
                165

<210>  16
<211>  125
<212>  PRT
<213>  Homo sapiens

<400>  16

Met Lys Lys Ser Gly Val Leu Phe Leu Leu Gly Ile Ile Leu Leu Val
1               5                   10                  15

Leu Ile Gly Val Gln Gly Thr Pro Val Val Arg Lys Gly Arg Cys Ser
            20                  25                  30

Cys Ile Ser Thr Asn Gln Gly Thr Ile His Leu Gln Ser Leu Lys Asp
        35                  40                  45

Leu Lys Gln Phe Ala Pro Ser Pro Ser Cys Glu Lys Ile Glu Ile Ile
    50                  55                  60

Ala Thr Leu Lys Asn Gly Val Gln Thr Cys Leu Asn Pro Asp Ser Ala
65                  70                  75                  80

Asp Val Lys Glu Leu Ile Lys Lys Trp Glu Lys Gln Val Ser Gln Lys
                85                  90                  95

Lys Lys Gln Lys Asn Gly Lys Lys His Gln Lys Lys Lys Val Leu Lys
            100                 105                 110

Val Arg Lys Ser Gln Arg Ser Arg Gln Lys Lys Thr Thr
        115                 120                 125

<210>  17
<211>  415
<212>  PRT
<213>  Homo sapiens

<400>  17

Met Glu Leu Arg Lys Tyr Gly Pro Gly Arg Leu Ala Gly Thr Val Ile
1               5                   10                  15

Gly Gly Ala Ala Gln Ser Lys Ser Gln Thr Lys Ser Asp Ser Ile Thr
            20                  25                  30

Lys Glu Phe Leu Pro Gly Leu Tyr Thr Ala Pro Ser Ser Pro Phe Pro
        35                  40                  45

Pro Ser Gln Val Ser Asp His Gln Val Leu Asn Asp Ala Glu Val Ala
    50                  55                  60

Ala Leu Leu Glu Asn Phe Ser Ser Ser Tyr Asp Tyr Gly Glu Asn Glu
65                  70                  75                  80

Ser Asp Ser Cys Cys Thr Ser Pro Pro Cys Pro Gln Asp Phe Ser Leu
                85                  90                  95

Asn Phe Asp Arg Ala Phe Leu Pro Ala Leu Tyr Ser Leu Leu Phe Leu
            100                 105                 110

Leu Gly Leu Leu Gly Asn Gly Ala Val Ala Ala Val Leu Leu Ser Arg
        115                 120                 125

Arg Thr Ala Leu Ser Ser Thr Asp Thr Phe Leu Leu His Leu Ala Val
    130                 135                 140

Ala Asp Thr Leu Leu Val Leu Thr Leu Pro Leu Trp Ala Val Asp Ala
145                 150                 155                 160

Ala Val Gln Trp Val Phe Gly Ser Gly Leu Cys Lys Val Ala Gly Ala
                165                 170                 175

Leu Phe Asn Ile Asn Phe Tyr Ala Gly Ala Leu Leu Leu Ala Cys Ile
            180                 185                 190

Ser Phe Asp Arg Tyr Leu Asn Ile Val His Ala Thr Gln Leu Tyr Arg
        195                 200                 205

Arg Gly Pro Pro Ala Arg Val Thr Leu Thr Cys Leu Ala Val Trp Gly
    210                 215                 220

Leu Cys Leu Leu Phe Ala Leu Pro Asp Phe Ile Phe Leu Ser Ala His
225                 230                 235                 240

His Asp Glu Arg Leu Asn Ala Thr His Cys Gln Tyr Asn Phe Pro Gln
                245                 250                 255

Val Gly Arg Thr Ala Leu Arg Val Leu Gln Leu Val Ala Gly Phe Leu
            260                 265                 270

Leu Pro Leu Leu Val Met Ala Tyr Cys Tyr Ala His Ile Leu Ala Val
        275                 280                 285

Leu Leu Val Ser Arg Gly Gln Arg Arg Leu Arg Ala Met Arg Leu Val
    290                 295                 300

Val Val Val Val Val Ala Phe Ala Leu Cys Trp Thr Pro Tyr His Leu
305                 310                 315                 320

Val Val Leu Val Asp Ile Leu Met Asp Leu Gly Ala Leu Ala Arg Asn
                325                 330                 335

Cys Gly Arg Glu Ser Arg Val Asp Val Ala Lys Ser Val Thr Ser Gly
            340                 345                 350

Leu Gly Tyr Met His Cys Cys Leu Asn Pro Leu Leu Tyr Ala Phe Val
        355                 360                 365

Gly Val Lys Phe Arg Glu Arg Met Trp Met Leu Leu Leu Arg Leu Gly
    370                 375                 380

Cys Pro Asn Gln Arg Gly Leu Gln Arg Gln Pro Ser Ser Ser Arg Arg
385                 390                 395                 400

Asp Ser Ser Trp Ser Glu Thr Ser Glu Ala Ser Tyr Ser Gly Leu
                405                 410                 415

<210>  18
<211>  178
<212>  PRT
<213>  Homo sapiens

<400>  18

Met His Ser Ser Ala Leu Leu Cys Cys Leu Val Leu Leu Thr Gly Val
1               5                   10                  15

Arg Ala Ser Pro Gly Gln Gly Thr Gln Ser Glu Asn Ser Cys Thr His
            20                  25                  30

Phe Pro Gly Asn Leu Pro Asn Met Leu Arg Asp Leu Arg Asp Ala Phe
        35                  40                  45

Ser Arg Val Lys Thr Phe Phe Gln Met Lys Asp Gln Leu Asp Asn Leu
    50                  55                  60

Leu Leu Lys Glu Ser Leu Leu Glu Asp Phe Lys Gly Tyr Leu Gly Cys
65                  70                  75                  80

Gln Ala Leu Ser Glu Met Ile Gln Phe Tyr Leu Glu Glu Val Met Pro
                85                  90                  95

Gln Ala Glu Asn Gln Asp Pro Asp Ile Lys Ala His Val Asn Ser Leu
            100                 105                 110

Gly Glu Asn Leu Lys Thr Leu Arg Leu Arg Leu Arg Arg Cys His Arg
        115                 120                 125

Phe Leu Pro Cys Glu Asn Lys Ser Lys Ala Val Glu Gln Val Lys Asn
    130                 135                 140

Ala Phe Asn Lys Leu Gln Glu Lys Gly Ile Tyr Lys Ala Met Ser Glu
145                 150                 155                 160

Phe Asp Ile Phe Ile Asn Tyr Ile Glu Ala Tyr Met Thr Met Lys Ile
                165                 170                 175

Arg Asn

<210>  19
<211>  288
<212>  PRT

<213>  Homo sapiens

<400>  19

Met Gly His Thr Arg Arg Gln Gly Thr Ser Pro Ser Lys Cys Pro Tyr
1               5                   10                  15

Leu Asn Phe Phe Gln Leu Leu Val Leu Ala Gly Leu Ser His Phe Cys
            20                  25                  30

Ser Gly Val Ile His Val Thr Lys Glu Val Lys Glu Val Ala Thr Leu
        35                  40                  45

Ser Cys Gly His Asn Val Ser Val Glu Glu Leu Ala Gln Thr Arg Ile
    50                  55                  60

Tyr Trp Gln Lys Glu Lys Lys Met Val Leu Thr Met Met Ser Gly Asp
65                  70                  75                  80

Met Asn Ile Trp Pro Glu Tyr Lys Asn Arg Thr Ile Phe Asp Ile Thr
                85                  90                  95

Asn Asn Leu Ser Ile Val Ile Leu Ala Leu Arg Pro Ser Asp Glu Gly
            100                 105                 110

Thr Tyr Glu Cys Val Val Leu Lys Tyr Glu Lys Asp Ala Phe Lys Arg
        115                 120                 125

Glu His Leu Ala Glu Val Thr Leu Ser Val Lys Ala Asp Phe Pro Thr
    130                 135                 140

Pro Ser Ile Ser Asp Phe Glu Ile Pro Thr Ser Asn Ile Arg Arg Ile
145                 150                 155                 160

Ile Cys Ser Thr Ser Gly Gly Phe Pro Glu Pro His Leu Ser Trp Leu
                165                 170                 175

Glu Asn Gly Glu Glu Leu Asn Ala Ile Asn Thr Thr Val Ser Gln Asp
            180                 185                 190

Pro Glu Thr Glu Leu Tyr Ala Val Ser Ser Lys Leu Asp Phe Asn Met
        195                 200                 205

Thr Thr Asn His Ser Phe Met Cys Leu Ile Lys Tyr Gly His Leu Arg
    210                 215                 220

Val Asn Gln Thr Phe Asn Trp Asn Thr Thr Lys Gln Glu His Phe Pro
225                 230                 235                 240

Asp Asn Leu Leu Pro Ser Trp Ala Ile Thr Leu Ile Ser Val Asn Gly
                245                 250                 255

Ile Phe Val Ile Cys Cys Leu Thr Tyr Cys Phe Ala Pro Arg Cys Arg
            260                 265                 270

Glu Arg Arg Arg Asn Glu Arg Leu Arg Arg Glu Ser Val Arg Pro Val
        275                 280                 285

<210>  20
<211>  217
<212>  PRT
<213>  Homo sapiens

<400>  20

Met Asp Pro Gln Cys Thr Met Gly Leu Ser Asn Ile Leu Phe Val Met
1               5                   10                  15

Ala Phe Leu Leu Ser Ala Asn Phe Ser Gln Pro Glu Ile Val Pro Ile
            20                  25                  30

Ser Asn Ile Thr Glu Asn Val Tyr Ile Asn Leu Thr Cys Ser Ser Ile
        35                  40                  45

His Gly Tyr Pro Glu Pro Lys Lys Met Ser Val Leu Leu Arg Thr Lys
    50                  55                  60

Asn Ser Thr Ile Glu Tyr Asp Gly Ile Met Gln Lys Ser Gln Asp Asn
65                  70                  75                  80

Val Thr Glu Leu Tyr Asp Val Ser Ile Ser Leu Ser Val Ser Phe Pro
                85                  90                  95

Asp Val Thr Ser Asn Met Thr Ile Phe Cys Ile Leu Glu Thr Asp Lys
            100                 105                 110

Thr Arg Leu Leu Ser Ser Pro Phe Ser Ile Glu Leu Glu Asp Pro Gln
        115                 120                 125

Pro Pro Pro Asp His Ile Pro Trp Ile Thr Ala Val Leu Pro Thr Val
    130                 135                 140

Ile Ile Cys Val Met Val Phe Cys Leu Ile Leu Trp Lys Trp Lys Lys
145                 150                 155                 160

Lys Lys Arg Pro Arg Asn Ser Tyr Lys Cys Gly Thr Asn Thr Met Glu
                165                 170                 175

Arg Glu Glu Ser Glu Gln Thr Lys Lys Arg Glu Lys Ile His Ile Pro
            180                 185                 190

Glu Arg Ser Asp Glu Ala Gln Arg Val Phe Lys Ser Ser Lys Thr Ser
        195                 200                 205

Ser Cys Asp Lys Ser Asp Thr Cys Phe
    210                 215

<210>  21
<211>  176
<212>  PRT
<213>  Homo sapiens

<400>  21

Met Arg Ile Phe Ala Val Phe Ile Phe Met Thr Tyr Trp His Leu Leu
1               5                   10                  15

Asn Ala Pro Tyr Asn Lys Ile Asn Gln Arg Ile Leu Val Val Asp Pro
            20                  25                  30

Val Thr Ser Glu His Glu Leu Thr Cys Gln Ala Glu Gly Tyr Pro Lys
        35                  40                  45

Ala Glu Val Ile Trp Thr Ser Ser Asp His Gln Val Leu Ser Gly Lys
    50                  55                  60

Thr Thr Thr Thr Asn Ser Lys Arg Glu Glu Lys Leu Phe Asn Val Thr
65                  70                  75                  80

Ser Thr Leu Arg Ile Asn Thr Thr Thr Asn Glu Ile Phe Tyr Cys Thr
                85                  90                  95

Phe Arg Arg Leu Asp Pro Glu Glu Asn His Thr Ala Glu Leu Val Ile
            100                 105                 110

Pro Glu Leu Pro Leu Ala His Pro Pro Asn Glu Arg Thr His Leu Val
        115                 120                 125

Ile Leu Gly Ala Ile Leu Leu Cys Leu Gly Val Ala Leu Thr Phe Ile
    130                 135                 140

Phe Arg Leu Arg Lys Gly Arg Met Met Asp Val Lys Lys Cys Gly Ile
145                 150                 155                 160

Gln Asp Thr Asn Ser Lys Lys Gln Ser Asp Thr His Leu Glu Glu Thr
                165                 170                 175

<210>  22
<211>  283
<212>  PRT
<213>  Homo sapiens

<400>  22

Met Ile Phe Leu Leu Leu Met Leu Ser Leu Glu Leu Gln Leu His Gln
1               5                   10                  15

Ile Ala Ala Leu Phe Thr Val Thr Val Pro Lys Glu Leu Tyr Ile Ile
            20                  25                  30

Glu His Gly Ser Asn Val Thr Leu Glu Cys Asn Phe Asp Thr Gly Ser
        35                  40                  45

His Val Asn Leu Gly Ala Ile Thr Ala Ser Leu Gln Lys Val Glu Asn
    50                  55                  60

Asp Thr Ser Pro His Arg Glu Arg Ala Thr Leu Leu Glu Glu Gln Leu
65                  70                  75                  80

Pro Leu Gly Lys Ala Ser Phe His Ile Pro Gln Val Gln Val Arg Asp
                85                  90                  95

Glu Gly Gln Tyr Gln Cys Ile Ile Ile Tyr Gly Val Ala Trp Asp Tyr
            100                 105                 110

Lys Tyr Leu Thr Leu Lys Val Lys Ala Ser Tyr Arg Lys Ile Asn Thr
        115                 120                 125

His Ile Leu Lys Val Pro Glu Thr Asp Glu Val Glu Leu Thr Cys Gln
    130                 135                 140

Ala Thr Gly Tyr Pro Leu Ala Glu Val Ser Trp Pro Asn Val Ser Val
145                 150                 155                 160

Pro Ala Asn Thr Ser His Ser Arg Thr Pro Glu Gly Leu Tyr Gln Val
                165                 170                 175

Thr Ser Val Leu Arg Leu Lys Pro Pro Pro Gly Arg Asn Phe Ser Cys
            180                 185                 190

Val Phe Trp Asn Thr His Val Arg Glu Leu Thr Leu Ala Ser Ile Asp
        195                 200                 205

Leu Gln Ser Gln Met Glu Pro Arg Thr His Pro Thr Trp Leu Leu His
    210                 215                 220

Ile Phe Ile Pro Phe Cys Ile Ile Ala Phe Ile Phe Ile Ala Thr Val
225                 230                 235                 240

Ile Ala Leu Arg Lys Gln Leu Cys Gln Lys Leu Tyr Ser Ser Lys Asp
                245                 250                 255

Thr Thr Lys Arg Pro Val Thr Thr Thr Lys Arg Glu Val Asn Ser Ala
            260                 265                 270

Val Asn Leu Asn Leu Trp Ser Trp Glu Pro Gly
        275                 280

<210>  23
<211>  372
<212>  PRT
<213>  Homo sapiens

<400>  23

Met Ala Arg Ala Met Ala Ala Ala Trp Pro Leu Leu Leu Val Ala Leu
1               5                   10                  15

Leu Val Leu Ser Trp Pro Pro Pro Gly Thr Gly Asp Val Val Val Gln
            20                  25                  30

Ala Pro Thr Gln Val Pro Gly Phe Leu Gly Asp Ser Val Thr Leu Pro
        35                  40                  45

Cys Tyr Leu Gln Val Pro Asn Met Glu Val Thr His Val Ser Gln Leu
    50                  55                  60

Thr Trp Ala Arg His Gly Glu Ser Gly Ser Met Ala Val Phe His Gln
65                  70                  75                  80

Thr Gln Gly Pro Ser Tyr Ser Glu Ser Lys Arg Leu Glu Phe Val Ala
                85                  90                  95

Ala Arg Leu Gly Ala Glu Leu Arg Asn Ala Ser Leu Arg Met Phe Gly
            100                 105                 110

Leu Arg Val Glu Asp Glu Gly Asn Tyr Thr Cys Leu Phe Val Thr Phe
        115                 120                 125

Pro Gln Gly Ser Arg Ser Val Asp Ile Trp Leu Arg Val Leu Ala Lys
    130                 135                 140

Pro Gln Asn Thr Ala Glu Val Gln Lys Val Gln Leu Thr Gly Glu Pro
145                 150                 155                 160

Val Pro Met Ala Arg Cys Val Ser Thr Gly Gly Arg Pro Pro Ala Gln
                165                 170                 175

Ile Thr Trp His Ser Asp Leu Gly Gly Met Pro Asn Thr Ser Gln Val
            180                 185                 190

Pro Gly Phe Leu Ser Gly Thr Val Thr Val Thr Ser Leu Trp Ile Leu
        195                 200                 205

Val Pro Ser Ser Gln Val Asp Gly Lys Asn Val Thr Cys Lys Val Glu
    210                 215                 220

His Glu Ser Phe Glu Lys Pro Gln Leu Leu Thr Val Asn Leu Thr Val
225                 230                 235                 240

Tyr Tyr Pro Pro Glu Val Ser Ile Ser Gly Tyr Asp Asn Asn Trp Tyr
                245                 250                 255

Leu Gly Gln Asn Glu Ala Thr Leu Thr Cys Asp Ala Arg Ser Asn Pro
            260                 265                 270

Glu Pro Thr Gly Tyr Asn Trp Ser Thr Thr Met Gly Pro Leu Pro Pro
        275                 280                 285

Phe Ala Val Ala Gln Gly Ala Gln Leu Leu Ile Arg Pro Val Asp Lys
    290                 295                 300

Pro Ile Asn Thr Thr Leu Ile Cys Asn Val Thr Asn Ala Leu Gly Ala
305                 310                 315                 320

Arg Gln Ala Glu Leu Thr Val Gln Val Lys Glu Gly Pro Pro Ser Glu
                325                 330                 335

His Ser Gly Thr Glu His Ala Ser Ala Ser Ala Asn Gly His Val Ser
            340                 345                 350

Tyr Ser Ala Val Ser Arg Glu Asn Ser Ser Ser Gln Asp Pro Gln Thr
        355                 360                 365

Glu Gly Thr Arg
    370

<210>  24
<211>  538
<212>  PRT
<213>  Homo sapiens

<400>  24

Met Ala Arg Ala Ala Ala Leu Leu Pro Ser Arg Ser Pro Pro Thr Pro
1               5                   10                  15

Leu Leu Trp Pro Leu Leu Leu Leu Leu Leu Leu Glu Thr Gly Ala Gln
            20                  25                  30

Asp Val Arg Val Gln Val Leu Pro Glu Val Arg Gly Gln Leu Gly Gly
        35                  40                  45

Thr Val Glu Leu Pro Cys His Leu Leu Pro Pro Val Pro Gly Leu Tyr
    50                  55                  60

Ile Ser Leu Val Thr Trp Gln Arg Pro Asp Ala Pro Ala Asn His Gln
65                  70                  75                  80

Asn Val Ala Ala Phe His Pro Lys Met Gly Pro Ser Phe Pro Ser Pro
                85                  90                  95

Lys Pro Gly Ser Glu Arg Leu Ser Phe Val Ser Ala Lys Gln Ser Thr
            100                 105                 110

Gly Gln Asp Thr Glu Ala Glu Leu Gln Asp Ala Thr Leu Ala Leu His
        115                 120                 125

Gly Leu Thr Val Glu Asp Glu Gly Asn Tyr Thr Cys Glu Phe Ala Thr
    130                 135                 140

Phe Pro Lys Gly Ser Val Arg Gly Met Thr Trp Leu Arg Val Ile Ala
145                 150                 155                 160

Lys Pro Lys Asn Gln Ala Glu Ala Gln Lys Val Thr Phe Ser Gln Asp
                165                 170                 175

Pro Thr Thr Val Ala Leu Cys Ile Ser Lys Glu Gly Arg Pro Pro Ala
            180                 185                 190

Arg Ile Ser Trp Leu Ser Ser Leu Asp Trp Glu Ala Lys Glu Thr Gln
        195                 200                 205

Val Ser Gly Thr Leu Ala Gly Thr Val Thr Val Thr Ser Arg Phe Thr
    210                 215                 220

Leu Val Pro Ser Gly Arg Ala Asp Gly Val Thr Val Thr Cys Lys Val
225                 230                 235                 240

Glu His Glu Ser Phe Glu Glu Pro Ala Leu Ile Pro Val Thr Leu Ser
                245                 250                 255

Val Arg Tyr Pro Pro Glu Val Ser Ile Ser Gly Tyr Asp Asp Asn Trp
            260                 265                 270

Tyr Leu Gly Arg Thr Asp Ala Thr Leu Ser Cys Asp Val Arg Ser Asn
        275                 280                 285

Pro Glu Pro Thr Gly Tyr Asp Trp Ser Thr Thr Ser Gly Thr Phe Pro
    290                 295                 300

Thr Ser Ala Val Ala Gln Gly Ser Gln Leu Val Ile His Ala Val Asp
305                 310                 315                 320

Ser Leu Phe Asn Thr Thr Phe Val Cys Thr Val Thr Asn Ala Val Gly
                325                 330                 335

Met Gly Arg Ala Glu Gln Val Ile Phe Val Arg Glu Thr Pro Asn Thr
            340                 345                 350

Ala Gly Ala Gly Ala Thr Gly Gly Ile Ile Gly Gly Ile Ile Ala Ala
        355                 360                 365

Ile Ile Ala Thr Ala Val Ala Ala Thr Gly Ile Leu Ile Cys Arg Gln
    370                 375                 380

Gln Arg Lys Glu Gln Thr Leu Gln Gly Ala Glu Glu Asp Glu Asp Leu
385                 390                 395                 400

Glu Gly Pro Pro Ser Tyr Lys Pro Pro Thr Pro Lys Ala Lys Leu Glu
                405                 410                 415

Ala Gln Glu Met Pro Ser Gln Leu Phe Thr Leu Gly Ala Ser Glu His
            420                 425                 430

Ser Pro Leu Lys Thr Pro Tyr Phe Asp Ala Gly Ala Ser Cys Thr Glu
        435                 440                 445

Gln Glu Met Pro Arg Tyr His Glu Leu Pro Thr Leu Glu Glu Arg Ser
    450                 455                 460

Gly Pro Leu His Pro Gly Ala Thr Ser Leu Gly Ser Pro Ile Pro Val
465                 470                 475                 480

Pro Pro Gly Pro Pro Ala Val Glu Asp Val Ser Leu Asp Leu Glu Asp
                485                 490                 495

Glu Glu Gly Glu Glu Glu Glu Glu Tyr Leu Asp Lys Ile Asn Pro Ile

500                 505                 510

Tyr Asp Ala Leu Ser Tyr Ser Ser Pro Ser Asp Ser Tyr Gln Gly Lys
        515                 520                 525

Gly Phe Val Met Ser Arg Ala Met Tyr Val
    530                 535

<210>  25
<211>  246
<212>  PRT
<213>  Homo sapiens

<400>  25

Met Ala Phe Ser Gly Ser Gln Ala Pro Tyr Leu Ser Pro Ala Val Pro
1               5                   10                  15

Phe Ser Gly Thr Ile Gln Gly Gly Leu Gln Asp Gly Leu Gln Ile Thr
            20                  25                  30

Val Asn Gly Thr Val Leu Ser Ser Ser Gly Thr Arg Phe Ala Val Asn
        35                  40                  45

Phe Gln Thr Gly Phe Ser Gly Asn Asp Ile Ala Phe His Phe Asn Pro
    50                  55                  60

Arg Phe Glu Asp Gly Gly Tyr Val Val Cys Asn Thr Arg Gln Asn Gly
65                  70                  75                  80

Ser Trp Gly Pro Glu Glu Arg Lys Thr His Met Pro Phe Gln Lys Gly
                85                  90                  95

Met Pro Phe Asp Leu Cys Phe Leu Val Gln Ser Ser Asp Phe Lys Val
            100                 105                 110

Met Val Asn Gly Ile Leu Phe Val Gln Tyr Phe His Arg Val Pro Phe
        115                 120                 125

His Arg Val Asp Thr Ile Ser Val Asn Gly Ser Val Gln Leu Ser Tyr
    130                 135                 140

Ile Ser Phe Gln Pro Pro Gly Val Trp Pro Ala Asn Pro Ala Pro Ile
145                 150                 155                 160

Thr Gln Thr Val Ile His Thr Val Gln Ser Ala Pro Gly Gln Met Phe
                165                 170                 175

Ser Thr Pro Ala Ile Pro Pro Met Met Tyr Pro His Pro Ala Tyr Pro

180                 185                 190

Met Pro Phe Ile Thr Thr Ile Leu Gly Gly Leu Tyr Pro Ser Lys Ser
        195                 200                 205

Ile Leu Leu Ser Gly Thr Val Leu Pro Ser Ala Gln Arg Cys Gly Ser
    210                 215                 220

Cys Val Lys Leu Thr Ala Ser Arg Trp Pro Trp Met Val Ser Thr Cys
225                 230                 235                 240

Leu Asn Thr Thr Ile Ala
                245

<210>  26
<211>  215
<212>  PRT
<213>  Homo sapiens

<400>  26

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Lys Lys Cys Ser Glu Arg
        35                  40                  45

Trp Lys Thr Met Ser Ala Lys Glu Lys Gly Lys Phe Glu Asp Met Ala
    50                  55                  60

Lys Ala Asp Lys Ala Arg Tyr Glu Arg Glu Met Lys Thr Tyr Ile Pro
65                  70                  75                  80

Pro Lys Gly Glu Thr Lys Lys Lys Phe Lys Asp Pro Asn Ala Pro Lys
                85                  90                  95

Arg Pro Pro Ser Ala Phe Phe Leu Phe Cys Ser Glu Tyr Arg Pro Lys
            100                 105                 110

Ile Lys Gly Glu His Pro Gly Leu Ser Ile Gly Asp Val Ala Lys Lys
        115                 120                 125

Leu Gly Glu Met Trp Asn Asn Thr Ala Ala Asp Asp Lys Gln Pro Tyr
    130                 135                 140

Glu Lys Lys Ala Ala Lys Leu Lys Glu Lys Tyr Glu Lys Asp Ile Ala

145                 150                 155                 160

Ala Tyr Arg Ala Lys Gly Lys Pro Asp Ala Ala Lys Lys Gly Val Val
                165                 170                 175

Lys Ala Glu Lys Ser Lys Lys Lys Lys Glu Glu Glu Glu Asp Glu Glu
            180                 185                 190

Asp Glu Glu Asp Glu Glu Glu Glu Glu Asp Glu Glu Asp Glu Asp Glu
        195                 200                 205

Glu Glu Asp Asp Asp Asp Glu
    210                 215

<210>  27
<211>  281
<212>  PRT
<213>  Homo sapiens

<400>  27

Met Asp Thr Thr Arg Tyr Arg Pro Trp Gly Arg Trp Val His Trp Ser
1               5                   10                  15

Arg Arg Pro Leu Phe Leu Ala Leu Ala Val Leu Val Thr Thr Val Leu
            20                  25                  30

Trp Ala Val Ile Leu Ser Ile Leu Leu Ser Lys Ala Ser Thr Glu Arg
        35                  40                  45

Ala Ala Leu Leu Asp Gly His Asp Leu Leu Arg Thr Asn Ala Ser Lys
    50                  55                  60

Gln Thr Ala Ala Leu Gly Ala Leu Lys Glu Glu Val Gly Asp Cys His
65                  70                  75                  80

Ser Cys Cys Ser Gly Thr Gln Ala Gln Leu Gln Thr Thr Arg Ala Glu
                85                  90                  95

Leu Gly Glu Ala Gln Ala Lys Leu Met Glu Gln Glu Ser Ala Leu Arg
            100                 105                 110

Glu Leu Arg Glu Arg Val Thr Gln Gly Leu Ala Glu Ala Gly Arg Gly
        115                 120                 125

Arg Glu Asp Val Arg Thr Glu Leu Phe Arg Ala Leu Glu Ala Val Arg
    130                 135                 140

Leu Gln Asn Asn Ser Cys Glu Pro Cys Pro Thr Ser Trp Leu Ser Phe

145                 150                 155                 160

Glu Gly Ser Cys Tyr Phe Phe Ser Val Pro Lys Thr Thr Trp Ala Ala
                165                 170                 175

Ala Gln Asp His Cys Ala Asp Ala Ser Ala His Leu Val Ile Val Gly
            180                 185                 190

Gly Leu Asp Glu Gln Gly Phe Leu Thr Arg Asn Thr Arg Gly Arg Gly
        195                 200                 205

Tyr Trp Leu Gly Leu Arg Ala Val Arg His Leu Gly Lys Val Gln Gly
    210                 215                 220

Tyr Gln Trp Val Asp Gly Val Ser Leu Ser Phe Ser His Trp Asn Gln
225                 230                 235                 240

Gly Glu Pro Asn Asp Ala Trp Gly Arg Glu Asn Cys Val Met Met Leu
                245                 250                 255

His Thr Gly Leu Trp Asn Asp Ala Pro Cys Asp Ser Glu Lys Asp Gly
            260                 265                 270

Trp Ile Cys Glu Lys Arg His Asn Cys
        275                 280